(19) **Europäisches Patentamt European Patent Office Office européen des brevets**

(11) **EP 4 183 384 A1**

(12) # EUROPEAN PATENT APPLICATION

(43) Date of publication:
**24.05.2023 Bulletin 2023/21**

(21) Application number: **21209513.7**

(22) Date of filing: **22.11.2021**

(51) International Patent Classification (IPC):
*A61K 9/00* (2006.01)  *B33Y 10/00* (2015.01)
*B33Y 70/00* (2020.01)  *A61K 31/519* (2006.01)
*A61P 25/18* (2006.01)

(52) Cooperative Patent Classification (CPC):
**A61K 9/0024; A61K 31/519; A61P 25/18;
B33Y 10/00; B33Y 70/00; B33Y 80/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Université de Mons
7000 Mons (BE)**
• **Université Libre de Bruxelles (ULB)
1050 Bruxelles (BE)**

(72) Inventors:
• **RAQUEZ, Jean-Marie
7000 Mons (BE)**
• **MANNINI, Giuseppe
7090 Braine-Le-Comte (BE)**
• **GOOLE, Jonathan
1950 Kraainem (BE)**
• **BENALI, Samira
59494 Petite-Forêt (FR)**

(74) Representative: **De Clercq & Partners
Edgard Gevaertdreef 10a
9830 Sint-Martens-Latem (BE)**

(54) **3D PRINTABLE PHARMACEUTICAL FORMULATIONS FOR MODIFIED RELEASE APPLICATIONS AND METHODS FOR THE MANUFACTURE THEREOF**

(57)     The application relates to the field of 3D printing objects for pharmaceutical applications. In particular, the present invention concerns formulations comprising an active pharmaceutical ingredient for 3D printing and methods of preparing them. Further, the present invention relates to 3D printed pharmaceutical dosage forms comprising an active pharmaceutical ingredient and methods of producing them. Also the use of the formulations or pharmaceutical dosage forms comprising an active pharmaceutical ingredient for use as a medicament is disclosed.

EP 4 183 384 A1

**Description**

**FIELD OF THE INVENTION**

The present invention relates to the field of 3D printing objects for modified-release pharmaceutical applications. In particular, the present invention concerns methods for preparing a pharmaceutical formulation or a printable filament comprising an active pharmaceutical ingredient (API). Further, the invention provides 3D printed pharmaceutical dosage forms and method for producing them. Further, the invention concerns the pharmaceutical formulation or 3D printable filament for use as a medicament.

**BACKGROUND OF THE INVENTION**

Nowadays, pharmaceutical companies are facing a real paradigm to develop efficient and scalable methods to meet the demand of personalized dosage forms. In this respect, three-dimensional (3D) printing represents a suitable technique to fulfil such requests, while enabling high manufacturing versatility at relatively low cost. For more than 30 years, the interest in 3D printing has been growing and is still being considered as a realistic scalable manufacturing technique.

Among the available 3D printing techniques, Fused Deposition Modelling (FDM) is currently regarded as the most low cost and easily accessible 3D printing technology. In a typical FDM process, a molten thermoplastic polymer filament is carried on by two rollers, molten in a high temperature heating block, extruded through a nozzle and finally deposited onto a build plate where the molten matter solidifies quickly. The printing head can move within the x- and y-axes whereas the platform, which can be thermostatically controlled, can move vertically on the z-axis, creating 3D structures layer-by-layer by fusing the layers together.

The use of polymers for 3D printing opens a wide range of possibilities to adapt the release profile of a loaded drug. For that purpose, most popular thermoplastic materials used in FDM and HME are described to be polylactic acid (PLA), polycaprolactone (PCL), polyvinyl alcohol (PVA), poly(lactic-coglycolic acid) (PLGA) and ethylene-vinyl acetate (EVA). However, such polymers need to be formulated and extruded as drug-loaded printable filaments before being 3D printed as drug delivery systems, for example by using Hot Melt Extrusion (HME) technology to produce the molten polymer filaments required for printing using FDM 3D printers.

**SUMMARY**

The present invention is at least in part based on the inventors' realization that both HME and 3D printing are potentially deleterious techniques for the loaded API, in particular an API in amorphous state, because of shear stress and the use of relatively high temperatures in these technologies. Amorphous drugs show higher reactivity and better solubility than their crystal counterparts, but on the other hand they are extremely unstable due to their increased reactivity. During HME and 3D printing, amorphous drug formulations are susceptible to recrystallizing, causing decreased solubility. In particular the high temperatures and shear stress present during HME and 3D printing negatively affect the stability of the API, finally leading to its degradation.

In the present application, methods are provided for the preparation of a pharmaceutical dosage form comprising an active pharmaceutical ingredient (API) and at least one thermoplastic polymer. The methods are particularly useful for APIs that are in an amorphous state, although they can be applied to all type of APIs.

In the present invention, the inventors developed a novel method for preparing a formulation or a printable filament comprising an active pharmaceutical ingredient (API) that can be used for 3D printing of a pharmaceutical dosage form. The method generally comprises melting a mixture comprising the API, at least one thermoplastic polymer and optionally at least one plasticizer at a temperature at which the API is substantially not decomposed. The method is particularly useful for API's in amorphous state which are more susceptible to degradation and instability caused by high temperatures and shear stress during melting processes, such as HME and 3D printing methods. Nevertheless, the methods of the invention can be used for all types of APIs. Hence, because of this preparation method the obtained formulation is particularly useful for HME and 3D printing, two deleterious techniques characterized by shear stress and high temperatures that might be harmful for an API, in particular an API in amorphous state.

The present application thus generally relates in a first aspect to a method for preparing a formulation comprising an active pharmaceutical ingredient (API), said method comprising melting a mixture comprising the API, at least one thermoplastic polymer and optionally one plasticizer, at a temperature at which the API is substantially not decomposed.

In some embodiments, the at least one thermoplastic polymer is a polyester, preferably a biodegradable polyester, more preferably poly(lactic) acid (PLA), poly(lactic-co-glycolic) acid (PLGA), polycaprolactone (PCL), polyhydroxybutyrate (PHB) or poly(3-hydroxy valerate) (PHV), such as amorphous PLA, amorphous PLGA, amorphous PCL, amorphous PHB, or amorphous PHV.

In some embodiments, the at least one thermoplastic polymer is ethylene vinyl acetate (EVA), also known as poly (ethylene-vinyl acetate) (PEVA), or polyvinyl alcohol (PVA), preferably the thermoplastic polymer is EVA and a further thermoplastic polymer having a melting temperature higher that the melting temperature of EVA.

In some embodiments, the at least one thermoplastic polymer present in the mixture or in the pre-formulation blend as disclosed herein is a combination of at least two different thermoplastic polymers; preferably a combination of thermoplastic polymer comprising polyester, preferably PLA, and a thermoplastic polymer comprising ethylene vinyl acetate.

In some embodiments, the plasticizer is a polyether, preferably the plasticizer is selected from the group consisting of polyethylene glycol (PEG), poloxamer (poly(ethylene glycol)-*block*-poly(propylene glycol)-*block*-poly(ethylene glycol)), and mixtures thereof. In some embodiments, the plasticizer is stearic acid. Preferably, the plasticizer is selected from the group consisting of polyethylene glycol 2000 (PEG2000), Poloxamer 188, Poloxamer 407, stearic acid, and mixtures thereof. More preferably the plasticizer is selected from the group consisting of PEG2000, Poloxamer 188, and mixtures thereof.

In particular preferred embodiments, the mixture comprises the API, PLA and EVA. In some other preferred embodiments, the mixture comprises the API, PLA and a PEG. In some other preferred embodiments, the mixture comprises the API, a PLA and a poloxamer.

In some embodiments, the melted mixture is solidified into a printable filament and the methods of the invention comprise modelling the printable filament into a pharmaceutical dosage form.

According to a further aspect, a formulation, preferably a pharmaceutical formulation, prepared according to a method as disclosed herein is provided.

According to a further aspect, a printable filament comprising a homogeneous mixture of an API and at least one thermoplastic polymer is provided, wherein the API in the printable filament is substantially not decomposed and/or wherein the printable filament is configured to melt at a temperature at which the API is substantially not decomposed.

In another aspect, a pharmaceutical dosage form is provided, preferably a 3D printed pharmaceutical dosage form, even more preferably a 3D printed modified release pharmaceutical dosage form, wherein the pharmaceutical dosage form is made from the formulation or from the printable filament as disclosed herein.

In another aspect, the formulation, printable filament or pharmaceutical dosage form as disclosed herein are for use as a medicament.

In a further aspect, a method for the treatment of a disease is disclosed, said method comprising administering the formulation, printable filament or pharmaceutical dosage form as disclosed herein to a subject in need thereof.

According to a further aspect, a method to prepare a printlet, preferably a modified-release printlet, is provided, said method comprising melting and printing the formulation or the printable filament as disclosed herein. Preferably, printing is performed with 3D printing; even more preferably with fused deposition modelling (FDM).

These and further aspects and preferred embodiments of the invention are described in the following sections and in the appended claims. The subject-matter of the appended claims is hereby specifically incorporated in this specification.

## BRIEF DESCRIPTION OF DRAWINGS

The following description of the figures of specific embodiments of the invention is merely exemplary in nature and is not intended to limit the present teachings, their application or uses.

**Fig. 1.** Dimensions of the implantable device (20 mm / 7 mm / 2. Mm (L/W/H) of an embodiment the invention

**Fig. 2.** DSC analysis of the different starting materials on the $2^{nd}$ heating.

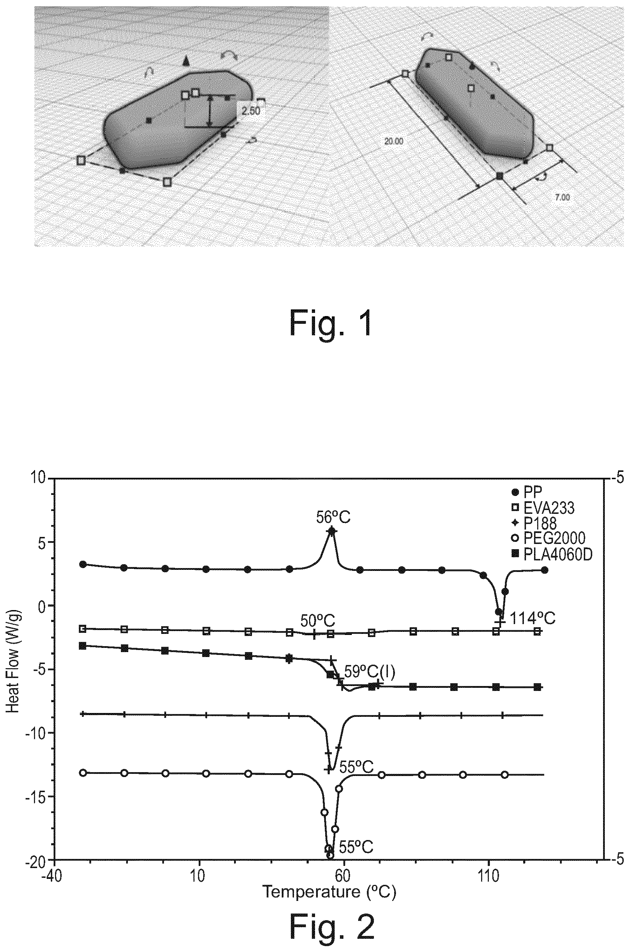

**Fig. 1.** TGA analysis of starting materials presented as weight loss (%) (UP) and its first derivative (DOWN).

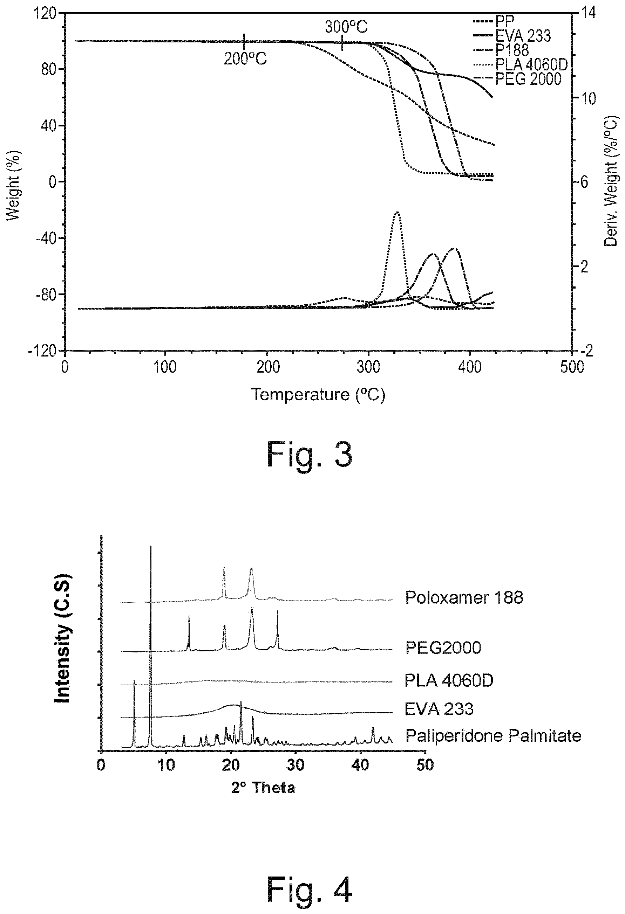

**Fig. 4.** XRD analysis of raw materials.

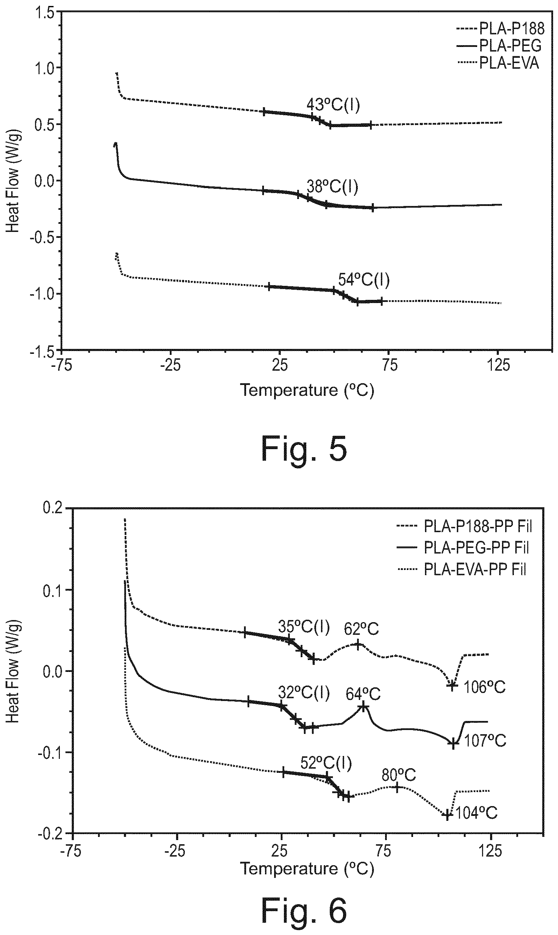

**Fig. 5.** DSC analysis of the pre-formulation according to certain embodiments of the invention on the first heating cycle.

**Fig. 6.** DSC analysis on the first heating cycle of the filaments according to certain embodiments of the invention after HME process: PLA_P188_PP, PLA_PEG_PP and PLA_EVA_PP.

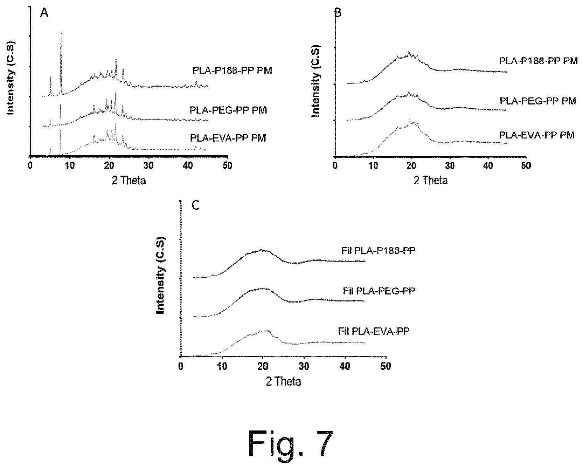

**Fig. 7.** XRD analysis on the formulations before (A) and after (B) cryogenic milling, and after HME process (C).

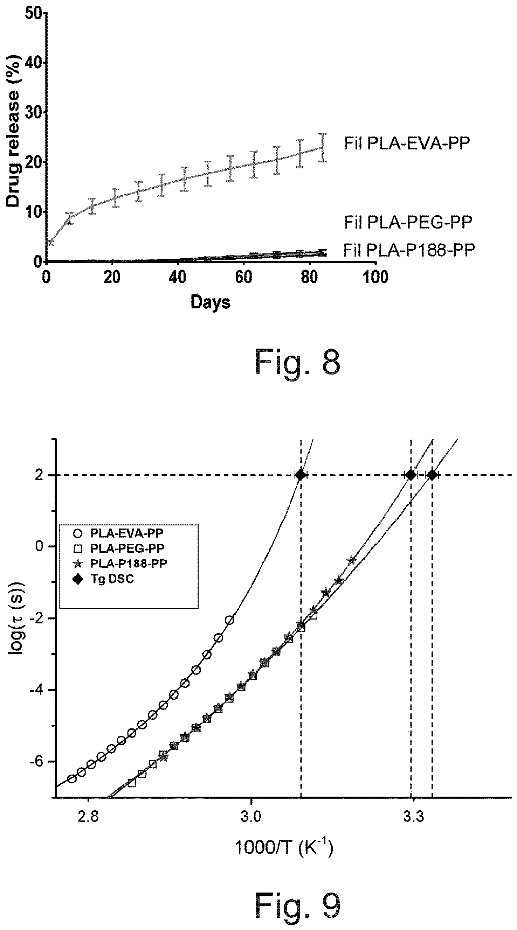

**Fig. 8.** Cumulative percentage of PP released over time from filaments according to certain embodiments of the invention based on PLA_P188_PP, PLA_PEG_PP and PLA_EVA_PP (mean $\pm$ SD, n=3).

**Fig. 9.** Relaxation time of PLA_EVA_PP, PLA_PEG_PP and PLA_P188_PP filaments according to certain embodiments of the invention as a function of temperature.

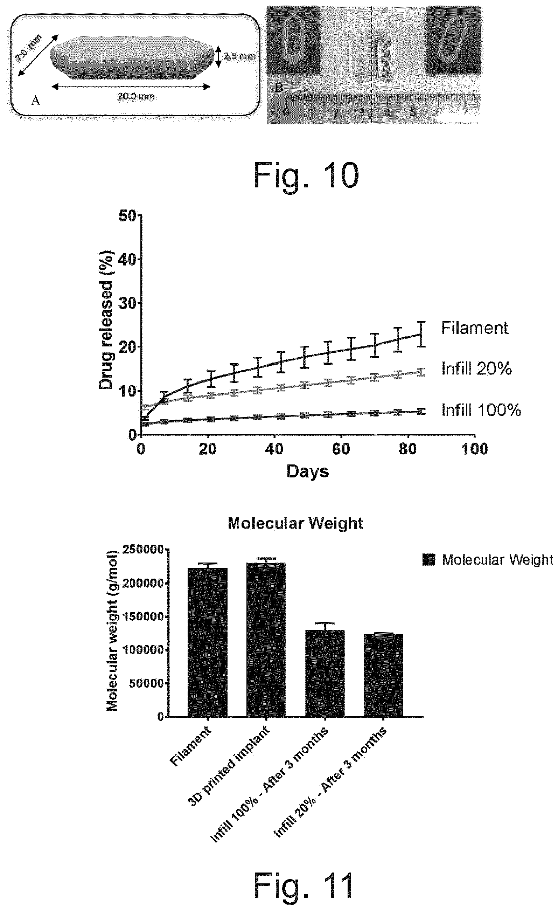

**Fig. 10.** Dimensions of the final implant (A) and photographs of 3D printed implants (B) according to certain embodiments of the invention.

**Fig. 11.** A) Cumulative percentage of PP released overtime from PLA_EVA_PP formulation (mean $\pm$ SD, n=3). B) Molecular weight (g/mol) of Filament, 3D printed implants and implants (100% and 20% infill) according to certain embodiments of the invention after 90 days based on PLA_EVA_PP (mean $\pm$ SD, n=3).

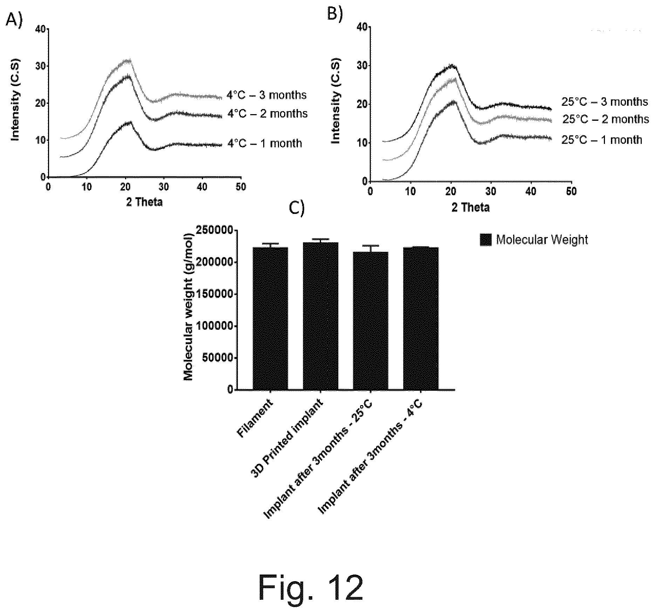

**Fig. 12.** XRD stability test on 3D printed PLA_EVA_PP based formulation according to certain embodiments of the invention on time and storage temperature A) stored at 4°C; B) stored at 25°C; C) GPC analysis of implants after 3 months stored at 25°C and 4°C (mean $\pm$ SD, n=3).

## DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Whereas the terms "one or more" or "at least one", such as one or more members or at least one member of a group of members, is clear per se, by means of further exemplification, the term encompasses *inter alia* a reference to any one of said members, or to any two or more of said members, such as, e.g., any $\geq 3$, $\geq 4$, $\geq 5$, $\geq 6$ or $\geq 7$ etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The present invention generally relates to novel methods for preparing formulations comprising an active pharmaceutical ingredient (API), wherein the formulations can be used for 3D printing technologies to obtain a pharmaceutical dosage form. The methods are particularly suitable for the preparation of pharmaceutical modified-release dosage forms, in particular printlets, which are printed pharmaceutical dosage forms. The methods as disclosed herein are typically characterized by a melting step of a mixture comprising the API, at least one thermoplastic polymer and optionally at least one plasticizer, wherein the melting is performed at a temperature at which the API is substantially not decomposed. The methods as disclosed herein can be applied for any type of API, but they are particularly useful for APIs that are in an amorphous state.

The inventors of the present application now developed novel methods for preparing a formulation comprising an API or for preparing a printable filament that can be used for 3D printing, in particular for 3D printing of a modified-release dosage form. More specifically, the inventors found that melting of a mixture comprising the API, at least one thermoplastic polymer and optionally at least one plasticizer at a lower temperature, in particular at a temperature at which the API is substantially not decomposed improved the API stability and API release in the final pharmaceutical dosage form, in particular after HME and/or 3D printing, for example 3D printing by FDM. In a preferred embodiment, the choice of the thermoplastic polymer allowed to decrease the processing temperature during melting, which resulted in a constant release of the API in the prepared pharmaceutical dosage form. In another preferred embodiment, and by selecting at least one solid plasticizer, the process temperature of the formulation could be further reduced, thereby improving the stability of the API.

In a first aspect, a method for preparing a formulation comprising an API is provided, said method comprises melting a mixture comprising the API, at least one thermoplastic polymer and optionally at least one plasticizer at a temperature at which the API is substantially not decomposed.

In another aspect, a method for preparing a formulation comprising an API is provided, said method comprises melting a mixture comprising the API and a pre-formulation blend comprising at least one thermoplastic polymer and optionally at least one plasticizer at a temperature at which the API is substantially not decomposed.

In the methods of the present application, melting of the mixture comprising the API, at least one thermoplastic polymer and optionally at least one plasticizer or melting of the mixture comprising the API and the pre-formulation blend occurs at a temperature at which the API is substantially not decomposed. Melting as used herein refers to the transition of a solid state into a liquid state. For example, when the mixture is a powder mixture, melting of the mixture results in transition of all compounds from a solid state into a liquid state. Or, when the mixture is a suspension, melting refers to the transition of the solid state compounds into a liquid state, resulting in a mixture wherein all compounds are in a liquid state.

Melting of the mixture as disclosed herein is performed at a temperature at which the API is substantially not decomposed. The temperature of the mixture will thus be set at a temperature at which the API is substantially not decomposed. Decomposition of the API as used herein refers to the state wherein the API has become unstable, denatured or degraded or wherein some part, such as at least 1%, at least 5%, or at least 10%, of the API is lost, which results in loss of the activity of the API or the generation of other adverse effects. Substantially not decomposed as used herein refers to the fact that a substantial amount of API will not be decomposed during melting or that a substantial amount of API remains in its original structure. A substantial amount of the API refers to at least 85 %; preferably at least 90 %, even more preferably at least 95 weight% of the total weight of the API that will not be decomposed, degraded or lost. For example, at least 90%, at least 91%, at least 92%, at least 93%, at least 94%, at least 95%, at least 96%; at least 97%, at least 98% or at least 99% of the total weight (w/w) of the API will not be decomposed; degraded or lost during melting of the mixture in any of the methods as used herein.

In order to measure the state of decomposition of an API, standard methods known to a skilled person can be used to evaluate the amount of the API that is lost or degraded or to evaluate the integrity of the API. For example, differential scanning calorimetry (DSC) and thermogravimetric analyses (TGA) can be used to measure the decomposition of the API whereas X-ray powder diffraction (XRD) can be used to characterize the structure of the API.

Preferably, melting is performed at a temperature at least 20°C lower, preferably at least 50°C lower, than the temperature at which the API starts to decompose. Melting is thus performed at a temperature at least 20°C, at least 30°C, at least 40°C or preferably at least 50°C lower than the temperature at which the API starts to decompose. For example, when an API starts to decompose at a temperature of 180°C, melting in the methods as intended herein is performed at a temperature of maximum about 160°C, or maximum about 150°C, or maximum about 140°C, preferably at a temperature of maximum about 130°C. For example, in such embodiments, when paliperidone palmitate is used as API, melting should ideally be performed at a temperature that is at least 20°C, preferably at least 50°C below 200°C, which is the temperature at which paliperidone palmitate shows a reduced thermal stability and weight loss, and thus starts to decompose. As used herein, the temperature at which the API starts to decompose is referred to as the temperature at which the weight or activity of the API is reduced with at least 1% after exposure to that temperature during a time range in between 30 minutes and 90 minutes, for example during 30 minutes, 60 minutes, 90 minutes, preferably during 60 minutes. In some embodiments, melting is performed at a temperature at most 20°C, preferably at most 10°C, higher than the melting temperature of the API. For example, melting is performed at most 20°C, at most 19°C, at most 18°C, at most 17°C, at most 16°C, at most 15°C, at most 14°C, at most 13°C, at most 12°C, at most 11°C, at most 10°C, higher than the melting temperature of the API. Thus, melting is performed at a temperature that is in between the melting temperature of the API and a temperature that is maximum 20°C higher than the melting temperature of the API, preferably a temperature that is maximum 10°C higher than the melting temperature. The melting temperature ($T_m$) of a substance as used herein refers to the melting point of that substance which is the temperature at which the substance changes from solid to liquid state. The melting temperature can for example be determined by using differential scanning calorimetry (DSC).

In a further embodiment, the method comprises melting a mixture comprising the API, the at least one thermoplastic polymer and optionally at least one plasticizer or melting a mixture comprising the API and the pre-formulation blend at a temperature at which the API and the thermoplastic polymer are substantially not decomposed. Similar to the temperature at which the API starts to decompose as described herein, the temperature at which the thermoplastic polymer starts to decompose is the temperature at which at least 1% of weight of the polymer is lost after an exposure time ranging between 30 minutes and 90 minutes; preferably after an exposure time of 60 minutes. Substantially not decomposed as used herein refers to the fact that a substantial amount of the API or thermoplastic polymer will not be decomposed during melting or that a substantial amount of API or thermoplastic polymer remains in its original structure. A substantial amount of the API or thermoplastic polymer refers to at least 85 %; preferably at least 90 %, even more preferably at least 95 weight% of the total weight (w/w) of the API or polymer that will not be decomposed, degraded or lost. For example, at least 90%, at least 91%, at least 92%, at least 93%, at least 94%, at least 95%, at least 96%; at least 97%, at least 98% or at least 99% of the total weight (w/w) of the API or polymer will not be decomposed; degraded or lost during melting.

The mixture as disclosed herein thus comprises at least one thermoplastic polymer. In some further embodiments, the mixture comprises a first thermoplastic polymer and a second thermoplastic polymer, and optionally at least one plasticizer. In some embodiments, the mixtures comprises at least one thermoplastic polymer, for example a first and second thermoplastic polymer, without the presence of a plasticizer. In still some further embodiments the mixture may comprise a first thermoplastic polymer, a second thermoplastic polymer and even a third thermoplastic polymer.

A thermoplastic polymer as used herein is a biodegradable and/or bioactive polymer having temperature-specific mechanical properties, preferably being solid at room temperature and becoming viscous liquid at increased temperature. Polymers go through several distinct phase transition points at particular temperatures. The most significant thermal transition points include melting and glass transition temperature. As used herein, the melting temperature ($T_m$) of a polymer is the temperature wherein the polymer changes from its physical state of a solid or its crystalline state into to a viscous flow or liquid state. Non-crystalline materials such as polymers also have glass transition temperature ($T_g$). The Tg is lower than the melting temperature and refers to the temperature where the polymer changes from a glass-like or hard-elastic, brittle state to a highly viscous or rubber-elastic, but not yet liquid, state.

In some embodiments, the mixture comprises a first thermoplastic polymer and a second thermoplastic polymer wherein the melting temperature of the second thermoplastic polymer is lower than that of the first thermoplastic polymer and wherein the melting temperature of the mixture is lower than that of the first thermoplastic polymer. Preferably, the melting temperature of the second thermoplastic polymer is at least 20°C, preferably at least 30°C lower than the melting temperature of the second thermoplastic polymer. For example, the melting temperature of the second thermoplastic polymer can be 20°C, 25°C, 30°C, 35°C, 40°C or even 50°C or more lower than the melting temperature of the first thermoplastic polymer. By combining a first and second thermoplastic polymer wherein the melting temperature of the second thermoplastic polymer is lower than the melting temperature of the first thermoplastic polymer the overall melting temperature of the mixture and the melting point of the API in the mixture is lowered, so that the mixture including the API can be melted and extruded at a lower temperature, thereby not substantially decomposing the API. For example, combination of two or more thermoplastic polymers in a mixture with an API may result in a reduction of the melting temperature of said API with at least 5°C, for example with 5°C, 10°C, 15°C or even more.

Thus, in some embodiments of the invention, a method for preparing a formulation comprising an API is provided wherein the method comprises melting a mixture comprising the API, at least a first and a second thermoplastic polymer wherein the melting temperature of the second thermoplastic polymer is lower than the melting temperature of the first thermoplastic polymer, and wherein the melting temperature of the mixture is lower than the melting temperature of the first thermoplastic polymer, and wherein melting of the mixture is performed at a temperature at which the API is substantially not decomposed.

The mixture that is melted during any of the methods disclosed herein may optionally comprise at least one plasticizer. A plasticizer, also referred to as a dispersant, refers to an additive that can increase the plasticity or decrease the viscosity of a composition or mixture. A plasticizer also reduces the melting temperature and glass transition temperature of a polymer. Thus, in some embodiments of the invention, a plasticizer is added to the mixture, thereby reducing the overall melting temperature of the mixture so that the mixture including the API can be melted and extruded at a lower temperature, thereby not substantially decomposing the API. For example, combination of one or more thermoplastic polymers with a plasticizer in a mixture with an API may result in a reduction of the melting temperature of said API in the mixture with at least 5°C, for example with 5°C, 10°C, 15°C or even more.

In some embodiments, the mixtures as disclosed herein thus comprise the API and at least one thermoplastic polymer. In some embodiments, the mixtures as disclosed herein comprise the API, a first thermoplastic polymer, and a second thermoplastic polymer. In some embodiments, the mixtures as disclosed herein thus comprise the API, at least one thermoplastic polymer, for example a first and a second thermoplastic polymer, and at least one plasticizer. In some preferred embodiments, the plasticizer is a solid plasticizer. The use of a solid plasticizer will reduce the risk of a leakage in the filament that is finally produced. Preferably, the at least one plasticizer is present in the mixture in between, and including, about 1 weight% (wt%) and 25 wt%, such as ranging from 1wt%, 2wt%, 3wt%, 4wt%, or 5 wt% to 20 wt%; 21 wt%, 22 wt%, 23 wt%, 24 wt% or 25 wt%, based on the total weight (w/w) of the mixture. Preferably the at least one plasticizer is present in the mixture in between, and including, about 5 weight% (wt%) and 25 wt% (w/w), even more preferably between about 5 wt% and 20 wt% (w/w), such as particularly preferably between about 5 wt% and 15 wt% (w/w), based on the combined weight of the at least one thermoplastic polymer and the at least one plasticizer in the mixture, such as for example about 5 wt%, 6 wt%, 7 wt%; 8 wt%, 9 wt%, 10 wt%, 11 wt%, 12 wt%, 13 wt%, 14 wt% or 15 wt% based on the combined weight (w/w) of the at least one thermoplastic polymer and the at least one plasticizer in the mixture. Thus, the mixture as disclosed herein may comprise for example 8 wt%, or 10 wt%, or 12 wt% of at least one plasticizer based on the combined weight (w/w) of the at least one thermoplastic polymer and the at least one plasticizer in the mixture.

The inventors further found that the glass transition temperature ($T_g$) of the mixtures comprising the API, the thermoplastic polymer and optionally the plasticizer, or of the mixtures comprising the API and the pre-formulation blend as disclosed herein affects the mobility and crystallization of the API, thereby affecting the stability of the API in the filament and in the final pharmaceutical dosage form. More specific, at a temperature that is above the $T_g$, an increase in molecular mobility can enhance the crystallization ability of the API, and reduces the temperature at which cold crystallization takes place. Thus, a mixture comprising an API and having a low $T_g$ will show an increased mobility as soon the temperature is above that Tg. For a mixture comprising an API and having a $T_g$ that is lower than the body temperature of a subject, i.e. lower than 37°C for a human subject, the mobility of the API will be increased, resulting in a faster crystallization of the API and a reduction of the API's release from the mixture or from a printable filament are formed thereof. Vice versa, for a mixture comprising an API and having a $T_g$ that is higher than the body temperature of a subject, i.e. higher than 37°C for a human subject, preferably having a $T_g$ that is above 40°C, preferably above 45°C, the mobility of the API is reduced, resulting in a slower crystallization of the API and an increase in the release of the API from the mixture or from a printable filament formed thereof. In some embodiments of the invention, the mixture comprising the API and at least one thermoplastic polymer, or the mixture comprising the pre-formulation blend comprising at least one thermoplastic polymer, thus has a glass transition temperature ($T_g$) that is above 37°C, preferably above 40°C, even more preferably above 45°C, such as a Tg of about 46°C, about 47°C, about 48°C, about 49°C, about 50°C, about 51°C, about 52°C, about 53°C, about 54°C, about 55°C, or higher.

In an aspect, a method for preparing a formulation comprising an API is provided wherein the method comprises melting a mixture comprising the API and a pre-formulation blend, said pre-formulation blend comprising at least one thermoplastic polymer and optionally at least one plasticizer, wherein the melting of the mixture occurs at a temperature at which the API is substantially not decomposed. The pre-formulation blend as disclosed herein thus comprises at least one thermoplastic polymer. In some embodiments, the pre-formulation blend further comprises at least one plasticizer. In some other embodiments, the pre-formulation blend comprises at least one thermoplastic polymer without the presence of a plasticizer. For example, the pre-formulation blend may comprise two different thermoplastic polymers without the presence of a plasticizer, or the pre-formulation blend may comprise one thermoplastic polymer and a plasticizer. In preferred embodiments, the pre-formulation blend comprises a first thermoplastic polymer, a second thermoplastic polymer, even more preferred wherein the melting temperature of the second thermoplastic polymer is lower than that of the first thermoplastic polymer and wherein the melting temperature of the mixture is lower than that of the first thermoplastic polymer. In some further embodiments, the pre-formulation blend has been obtained by melting and allowing to solidify a mixture comprising the at least one thermoplastic polymer and optionally the at least one plasticizer. Thus, a mixture comprising the at least one thermoplastic polymer, such as the first and second thermoplastic polymer, and optionally at least one plasticizer, will be melted and allowed to solidify, thereby forming the pre-formulation blend.

Even further, in some embodiments, said pre-formulation blend has been obtained by hot melt extrusion (HME) of the mixture comprising the at least one thermoplastic polymer, such as the first and second thermoplastic polymer, and optionally the at least one plasticizer.

In the context of the invention, the mixture comprising the API, the at least one thermoplastic polymer and optionally the at least one plasticizer or the mixture comprising the API and the pre-formulation blend can be a powder or a suspension comprising these compounds. In preferred embodiments, the mixture is a powder, i.e. a solid mixture wherein each of the compounds is present in a solid state as a powder. In some other embodiments, the mixture is a suspension, i.e. a mixture comprising compounds in solid state and in liquid state. For example, in such suspension mixture, the API can be present in a solid state, for example as a powder, whereas the at least one thermoplastic polymer and optionally the at least one plasticizer are present in a liquid state, for example as a solution. In some embodiments, the mixture is a homogeneous mixture, such as a homogeneous powder mixture, wherein each compound is homogeneously present in the mixture so that the powder mixture has a uniform composition.

In some embodiments, the API, the at least one thermoplastic polymer, such as the first and second thermoplastic polymer, and optionally a plasticizer are intimately mixed in the mixture thereby forming an intimate mixture showing a close union of the different particles or elements, more preferably wherein said intimate mixture is a powder. In certain embodiments, the intimate mixture has been obtained by milling, preferably cryogenic milling, of the API, the at least one thermoplastic polymer, such as the first and second thermoplastic polymer, and optionally the plasticizer. In some embodiments of the application, the API and the pre-formulation blend as disclosed herein are intimately mixed thereby forming an intimate mixture thereof, preferably wherein the intimate mixture is a powder, even more preferably wherein the intimate mixture has been obtained by milling, preferably cryogenic milling, of the API and the pre-formulation blend.

In some embodiments, the methods as disclosed herein further comprise allowing the melted mixture comprising the API, the at least one thermoplastic polymer and optionally the at least one plasticizer, or the mixture comprising the API and the pre-formulation blend to solidify. In some further embodiments, the melted mixture has solidified into a filament, more preferably into a printable filament. The melt solidification can for example occur via cooling or evaporation.

In some embodiments, the methods for preparing a formulation comprising an API as disclosed herein comprise hot melt extrusion of the mixture comprising the API and the at least one thermoplastic polymer and optionally the at least one plasticizer, or of the mixture comprising the API and the pre-formulation blend that comprises the at least one thermoplastic polymer and optionally the at least one plasticizer.

In some further embodiments, melting of the mixture comprising the API, the at least one thermoplastic polymer and optionally the at least one plasticizer, or melting of the mixture comprising the API and the pre-formulation blend, is performed at a temperature at most 30°C, preferably at most 20°C, more preferably at most 10°C, higher than the melting temperature of the API. Thus, in such embodiments, melting of the mixture should be performed at a temperature that is for example maximum 30°C, or maximum 25°C, or maximum 20°C, or maximum 15°C, or maximum 12°C higher than the melting temperature of the API. Preferably, melting of the mixture is performed at a temperature that is maximum 10°C higher than the melting temperature of the API, such as for example 10°C, 8°C, 6°C higher than the melting temperature of the mixture. Melting of the mixture can also be performed at the melting temperature of the API. More specific, and as an example, when paliperidone palmitate is used as API, melting is performed at most 30°C, preferably at most 20°C; even more preferably at most 10°C higher than 115 °C, which is the melting temperature of paliperidone palmitate.

There may be more than one thermoplastic polymer comprised in the mixture, the pre-formulation blend and the formulation of the present application, such as two, three, four or more thermoplastic polymers. In some embodiments, there are two different thermoplastic polymers present in the mixture, pre-formulation blend or formulation of the present application, without the presence of a plasticizer. In some other embodiments, there are one or two different thermoplastic polymers in the mixture, pre-formulation blend or formulation in the presence of a plasticizer.

The at least one thermoplastic polymer as disclosed herein may be a thermoplastic polyester, preferably a biodegradable thermoplastic polyester. In some preferred embodiments, the thermoplastic polymer is a polyester, preferably a biodegradable polyester. In still some further embodiments, the thermoplastic polymer may be poly(lactic) acid (PLA), poly(lactic-co-glycolic) acid (PLGA), polycaprolactone (PCL), polyhydroxybutyrate (PHB) and poly(3-hydroxy valerate) (PHV), such as amorphous PLA, amorphous PLGA, amorphous PCL, amorphous PHB, and amorphous PHV. The thermoplastic polymer may also be ethylene vinyl acetate (EVA), also known as poly (ethylene-vinyl acetate) (PEVA), or polyvinyl alcohol (PVA), preferably the thermoplastic polymer is EVA Even more preferably, the at least one thermoplastic polymer is PLA, preferably amorphous PLA or a polymer mixture comprising amorphous PLA. In some other embodiments, the thermoplastic polymer is a mixture of PLA and EVA.

PLA is a biodegradable high-performance thermoplastic suitable for use in medical 3D printing technologies. PLA may also be mixed with other thermoplastic polymers in any concentration and/or proportion, preferably with one or more thermoplastic polymers as listed herein. In some preferred embodiments, PLA is mixed with EVA.

In some embodiments, the mixture comprises two types of thermoplastic polymers, preferably EVA and a further thermoplastic polymer having a melting temperature higher than EVA.

The plasticizer as intended herein is preferably in a solid form. The plasticizer is preferably a polyether. The plasticizer may be stearic acid. Examples of plasticizers include polyethylene glycol (PEG), poloxamer (poly(ethylene glycol)-*block*-poly(propylene glycol)-*block*-poly(ethylene glycol)), stearic acid, and mixtures thereof. In some embodiments, the plasticizer is polyethylene glycol (PEG), also known as polyethylene oxide (PEO) or polyoxyethylene. Different types of PEG, each with a different molecular weight, can be used; such as for example PEG600 with an average molecular weight of 600, or PEG1000 with an average molecular weight of 1000, or PEG2000 with an average molecular weight of 2000 g/mol. In some embodiments, the plasticizer is a poloxamer, which is a nonionic triblock copolymer composed of a central hydrophobic chain of polyoxypropylene flanked by two hydrophilic chains of polyoxyethylene (poly(ethylene oxide)). Examples of poloxamers, each with a different molecular weight include, but are not limited to poloxamer 188 (2-2(propoxypropoxy)ethanol) with an average molecular weight 8400 g/mol, and poloxamer 407 with an average molecular weight 12500 g/mol.

In some embodiments, the mixture comprising the API and at least one thermoplastic polymer, or the mixture comprising the API and the pre-formulation blend as disclosed herein may comprise between 50 wt% and 99 wt% of the at least one thermoplastic polymer, preferably between 60 wt% and 99 wt%, even more preferably between 70 wt% and 99 wt%, of the total weight of the mixture (w/w). Thus, in some embodiments, the total weight of the first and second thermoplastic polymer in the mixture is between 50 wt% and 99 wt%, preferably between 60 wt% and 99 wt%, even more preferably between 70 wt% and 99 wt% based on the total weight of the mixture (w/w).

In some embodiments, the mixture comprises between 50 wt% and 99 wt% of PLA, preferably between 60 wt% and 99 wt% of PLA of the total weight of the mixture (w/w). In some other embodiments, the mixture comprises PLA in combination with one or more of the other listed thermoplastic polymers, such as EVA, such that the total amount of thermoplastic polymers in the mixture is between 50 weight% (wt%) and 99 wt%, preferably between 60 wt% and 99 wt%, even more preferably between 70 wt% and 99 wt% of the total weight of the mixture (w/w).

In some embodiments, the mixture may comprise between 60 wt% and 85 wt% of PLA, preferably between 60 wt% and 75 wt% of PLA, in combination with between 5 wt% and 30 wt% of a second thermoplastic polymer, such as EVA, preferably between 10 wt% and 20 wt% of a second thermoplastic polymer, based on the total weight of the mixture (w/w). For example, the mixture may comprise about 65 wt% of PLA in combination with 20 wt% of a second thermoplastic polymer, such as EVA, or 70 wt% of PLA in combination with about 20 wt% of a second thermoplastic polymer, such as EVA; or about 72 wt% of PLA in combination with about 18 wt% of a second thermoplastic polymer, such as EVA, based on the total weight of the mixture (w/w).

The mixture or the pre-formulation blend may comprise more than one thermoplastic polymer. In case two different types of thermoplastic polymers are present in the mixture or pre-formulation blend, the first thermoplastic polymer may be present in between 1 - 99 wt% and the second thermoplastic polymer may be present in between 99 - 1 wt%, relative to the total weight (w/w) of the thermoplastic polymer. Preferably, the first thermoplastic polymer may be present in between 20 -95 wt% and the second thermoplastic polymer may be present in between 80 - 5 wt%, relative to the total weight (w/w) of the thermoplastic polymer. Even more preferably, the first thermoplastic polymer may be present in between 50 - 90 wt% and the second thermoplastic polymer may be present in between 10 -50 wt%, related to the total weight (w/w) of the thermoplastic polymer. For example, the mixture or pre-formulation blend may comprise 50 wt% of a first thermoplastic polymer in combination with 50 wt% of a second thermoplastic polymer, relative to the total weight (w/w) of the polymer. Or, the mixture or pre-formulation blend may comprise 60 wt%, 70 wt%; 80 wt% or 90 wt% of a first thermoplastic polymer in combination with 40 wt%, 30 wt%, 20 wt% or 10 wt% respectively of a second thermoplastic polymer, relative to the total weight (w/w) of the polymer.

In some embodiments, the mixture as disclosed herein may comprise at least one plasticizer. In some embodiments, the plasticizer is present in the mixture in an amount sufficient to reduce the melting temperature of the API in the mixture to a temperature at which the API is substantially not decomposed, for example to reduce the melting temperature of the API in the mixture with at least 5°C. In some embodiments, the at least one plasticizer is present between 2 to 25 wt% of at least one plasticizer, preferably between 5 wt% and 20 wt% of at least one plasticizer, of the total weight of the mixture (w/w). In some embodiments, the mixture as disclosed herein comprises between 50 wt% and 95 wt% of at least one thermoplastic polymer in combination with 2 wt% to 25 wt% of at least one plasticizer, of the total weight of the mixture (w/w). For example, the mixture as disclosed herein may comprise 7 wt%, 8 wt%, 9 wt%, 10 wt%, 11 wt%, 12 wt%, 13 wt%, 14 wt% or 15 wt% of at least one plasticizer, based on the total weight of the mixture (w/w).

The mixture or the pre-formulation blend may thus comprise one or more thermoplastic polymers and at least one plasticizer. The plasticizer may be present in between 1 - 30 wt% relative to the total weight of the thermoplastic polymer and plasticizer. Preferably, the plasticizer may be present in between 2 - 25 wt% relative to the total weight (w/w) of the thermoplastic polymer and plasticizer. For example, the mixture or pre-formulation blend may comprise 5 wt%, 7 wt%; 10 wt%, 12 wt% or 15 wt% of a plasticizer or a combination of plasticizers, in combination with 95 wt%, 93 wt%, 90 wt%, 88 wt% or 85 wt% respectively of a thermoplastic polymer or combination of thermoplastic polymers, relative to the total weight (w/w) of the thermoplastic polymer and plasticizer.

In some preferred embodiments, the mixture comprises, consists substantially of or consists of the API in combination with PLA and EVA. In some embodiments, the API in the mixture comprising PLA and EVA may vary from 1 wt% to 20 wt%, preferably from 2 wt% to 20 wt%, such as for example 2 wt%, 5 wt%, 10 wt%, or 15 wt%, of the total weight (w/w) of the mixture. In some embodiments, the mixture comprises, consists substantially of or consists of the API in combination with 60 wt% to 98 wt%, preferably 60 wt% to 90 wt%, more preferably 60 to 80 wt%, of PLA in combination with 1 wt% to 39 wt%, preferably 3 wt% to 30 wt%, more preferably 5 wt% to 20 wt% of EVA based on the total weight of the mixture (w/w). For example, the mixture may comprise, consist substantially of or consist of about 65 wt% of PLA in combination with 20 wt% of EVA, or 70 wt% of PLA in combination with about 20 wt% of EVA, or about 72 wt% of PLA in combination with about 18 wt% of EVA, based on the total weight of the mixture (w/w). In some other embodiments, the mixture may comprise, consist substantially of or consist of about 60 wt% of PLA in combination with 20 wt% of EVA, or 60 wt% of PLA in combination with about 25 wt% of EVA, or about 75 wt% of PLA in combination with about 15 wt% of EVA, based on the total weight of the mixture (w/w). Possible combinations of the API and PLA with EVA in the mixture are presented in Table 1.

**Table 1: Examples of mixtures comprising an API, PLA and EVA according to some embodiments of the invention. The PLA and EVA can be pre-formulated in a pre-formulation blend. Wt% for the API, PLA and EVA are indicated relative to the total weight of the mixture (w/w).**

| Example | API (wt%) | PLA (wt%) | EVA (wt%) |
|---------|-----------|-----------|-----------|
| 1 | 2 | 90 | 8 |
| 2 | 2 | 85 | 13 |
| 3 | 2 | 80 | 18 |
| 4 | 2 | 78 | 20 |
| 5 | 5 | 90 | 5 |
| 6 | 5 | 85 | 10 |
| 7 | 5 | 80 | 15 |
| 8 | 5 | 75 | 20 |
| 9 | 10 | 65 | 25 |
| 10 | 10 | 70 | 20 |
| 11 | 10 | 72 | 18 |
| 12 | 10 | 75 | 15 |
| 13 | 12 | 85 | 3 |
| 14 | 12 | 80 | 8 |
| 15 | 12 | 75 | 13 |
| 16 | 12 | 70 | 18 |

Where the mixture comprises at least one plasticizer, preferably at least one solid plasticizer, said at least one plasticizer is preferably present in the mixture between about 1 wt% and 30 wt%, preferably between about 5 wt% and 25 wt% (w/w) based on the combined weight of the at least one thermoplastic polymer and the at least one plasticizer in the mixture. Even more preferred, the at least one plasticizer is present in the mixture between about 5 wt% and 20 wt% (w/w); more specific between about 5 wt% and 15 wt% (w/w) based on the combined weight (w/w) of the at least one thermoplastic polymer and the at least one plasticizer in the mixture. In some preferred embodiments, the plasticizer is PEG, preferably PEG2000. In some other preferred embodiments, the plasticizer is a poloxamer, preferably Poloxamer 188. Thus, in some embodiments, the mixture comprises the API in combination with between 50 wt% to 97 wt% preferably between 60 wt% to 97 wt%, even more preferably between 70 wt% to 97 wt%, of at least one thermoplastic polymer, preferably PLA, and between 2 wt% to 25 wt% of a plasticizer such as PEG or a poloxamer, based the combined weight (w/w) of the mixture. In some preferred embodiments, the mixture comprises the API in combination with PLA and PEG, preferably PEG2000. More specifically, the mixture comprises the API in combination with 70 wt% to 97 wt% of PLA in combination with 2 wt% to 25 wt% PEG, preferably PEG2000, based on the total weight of the mixture (w/w). In some other preferred embodiments, the mixture comprises PLA and a poloxamer; preferably Poloxamer 188. More specifically, the mixture comprises the API in combination with 70 wt% to 97 wt% of PLA and 2 wt% to 25 wt% of poloxamer, preferably Poloxamer 188, based on the total weight of the mixture (w/w). Thus, the mixture may comprise for example 5 wt%, 6 wt%, 7 wt%, 8 wt%, 9 wt%, 10 wt%, 11 wt%, 12 wt%, 13 wt%, 14 wt% or 15 wt% of a PEG, preferably PEG2000, or of a poloxamer, preferably Poloxamer 188.

The active pharmaceutical ingredient (API) as used herein refers to a substance or compound in a pharmaceutical formulation that is biologically active and is meant to produce a desired therapeutic effect in the body. Other terms such as active compound, active substance, or active constituent and active ingredient indicate a similar meaning and purpose and may therefore be used interchangeably herein. The APIs may be categorized into different classes according to various classification systems which differentiate APIs based on their intended purpose, mechanism of action and/or physical parameters such as solubility. The present application is by no means limited to a particular API type of class. However, in view of the intended effect of providing modified-release pharmaceutical applications, the API will preferably be an API that benefits from such modified release. In some preferred embodiments, the API is selected from the Biopharmaceutics Classification System (BCS) class II drugs which are high permeable drugs showing low solubility. Examples of drugs that belong to the BCS Class II are paliperidone (also known as risperidone), paliperidone palmitate, ibuprofen, ketoprofen, carvedilol, ketoconazole, fenofibrate, danazol, griseofulvin, metoprolol, nifedipine, clopidogrel, glibenclamide, carbamazepine, haloperidol, itraconazole, praziquantel, diclofenac, indomethacin, piroxicam, naproxen, lovastatin, lansoprazole, atorvastatin, glipizide and ofloxacin. In some embodiments, the API is thus a BCS class II drug or derivative, such as ester or fatty acid ester, thereof. In a preferred embodiment, the API is paliperidone or paliperidone palmitate (PP). In a further preferred embodiment, the API is paliperidone palmitate.

Further, and also in view of the specific features of the invention, the API is an API that is at least partly in amorphous state or an API in crystalline form. Preferably, the API is at least party in amorphous state, preferably at least 50%, even more preferably at least 70%, of the API is in amorphous state. Also further, and in view of the specific features of the invention, the API is a heat-sensitive API. Thus, in some most preferred embodiments, the API is a heat-sensitive API in amorphous state.

In some embodiments, the API is present in an amount of between 1 wt% and 20 wt%, preferably between 5 wt% and 15 wt%; such as between 7 wt% and 12 wt%, of the total weight of the mixture (w/w). For example, the API is present in an amount of about 5 wt%, 6 wt%, 7 wt%, 8 wt%, 9 wt%, 10 wt%, 11 wt%, 12 wt%, 13 wt%, 14 wt%, or 15 wt%,of the total weight of the mixture (w/w).

Modified-release pharmaceutical application as used herein refers to a form of solid dosage wherein release of the API is modified. It is non-immediate-release form. Release of the API may be delayed to reach a specific timing and/or target; this may also be referred to as a delayed-release application. Release of the API may also be extended over a prolonged period of time to prolong and/or maintain a specific concentration of available API; this may also be referred to as a sustained-release application. Additionally, the modified-release may be a combination of delayed, timed, targeted, prolonged, extended and/or sustained release.

In some embodiments, the methods as disclosed herein are characterized in that the melted mixture has solidified into a printable filament. Preferably, the method further comprises modelling of said printable filament into a pharmaceutical dosage from. In preferred embodiments, said modelling is performed using fused deposition modelling (FDM). The modelling thus results in the formation of a pharmaceutical dosage form, even more preferred a modified-release dosage form, such as a delayed-release dosage form or a sustained-release dosage form. Modified-release as used herein refers to a form of dosage wherein release of the API is modified; it is a non-immediate release form. Released of the API may be delayed to reach a specific timing and/or target. This may also be referred to as a delayed-release application. Release of the API may also be extended over a prolonged time period to prolong and/or maintain a specific concentration of available API. This may also be referred to a sustained-release. Additionally, the modified-release may be a combination of delayed, timed, targeted, prolonged, extended and/or sustained release. For example, the modified-release dosage form is a dosage form wherein the API is released over a prolonged time interval, such as over at least 2 hours to at most 48 hours, preferably over at least 6 hours to at most 24 hours. The modified-release dosage form can also be used to release the API over a much longer time interval, such as from 10 days to 100 days, or from 100 days to 300 days, or even more. For example, the modified-release dosage form is a dosage form wherein the API is released over a time interval in between 30 days to 100 days, for example, 30 days, 60 days, or 90 days; or wherein the API is released over a time interval in between 100 days and 500 days, such as for example 100 days, 200 days, 300 days, or even 3 months, 6 months, or 1 year. The modified-release dosage form as used herein can be developed such that the release of the API is gradually and continuously spread over time to ensure a continuous release of the API over the complete release period. For example, the modified-release dosage form may release the API at a constant release rate of in between 5% and 50% over time, preferably in between 10% and 30% over time, such as for example a constant release rate of 10%, 15%, or 20% over time.

For example, the modelling thus results in the formation of a printlet, such as an implantable printlet; even more preferred an implantable modified-release printlet.

As used herein, a "printlet" refers to any printed shape suitable for pharmaceutical use. A printlet is thus a printed pharmaceutical dosage form. In a further embodiment, a printlet can be in the form of a tablet.

In an aspect of the invention, a formulation prepared according to any of the methods disclosed herein is disclosed. Even further, said formulation is a pharmaceutical formulation comprising an API.

In another aspect, a printable filament is disclosed, said filament comprises a mixture of an API and at least one thermoplastic polymer, wherein the filament is configured to melt at a temperature at which the API is substantially not decomposed. In some further embodiments, the mixture comprises an API, at least one thermoplastic polymer and a plasticizer. In still some further embodiments, the homogeneous mixtures comprises an API, a first thermoplastic polymer, a second thermoplastic polymer and optionally a plasticizer. The API, thermoplastic polymers and plasticizer can be selected from any of the lists disclosed herein. In some preferred embodiments, the filaments comprises a homogeneous mixture of an API, PLA, and one or more of PEG, EVA or a poloxamer, such as Poloxamer 188. As said the filament is configured to melt at a temperature at which the API is substantially not decomposed. In some further embodiments, and similar to the mixture as disclosed herein, the filament is configured to melt at a temperature that is at least 20°C; preferably at least 50°C, lower than the temperature at which the API begins to decompose. In some embodiments, the filament is configured to melt at a temperature that is at most 20°C; preferably at most 10°C, higher than the temperature of the API.

Further aspects of the invention also include a pharmaceutical dosage form, preferably a 3D printed pharmaceutical dosage form such as a pharmaceutical printlet, even more preferably a 3D printed modified release pharmaceutical dosage form or printlet made from the formulation or from the printable filament as disclosed in any of the embodiments described herein. In some embodiments, the 3D printed pharmaceutical dosage form presents with an infill in between 1% and 100%, preferably in between 2% and 40%; even more preferably in between 5% and 25%, such as for example an infill of 5%, 6%, 7%, 8%, 9%, 10%, 11%, 12%, 13%, 14%, 15%, 16%, 17%, 18%, 19% or 20%.

The methods and formulation as disclosed herein may particularly be useful in 3D printing technologies. The formulations and printable filaments may therefore be adapted to the requirements for the 3D printing technology. In case the 3D printing technology is a 3D printing technology that uses printable filaments, the formulation is preferably used for producing a 3D printable filament comprising the formulation. The 3D printable filament may be for instance produced using hot melt extrusion (HME). In case the 3D printing technology is fused-deposition modelling (FDM) 3D printing, the formulation is preferably used for producing an FDM compatible 3D printable filament comprising the formulation.

The 3D printing may be performed by means of any 3D printing technology known in the art. It is understood that the skilled person will be capable of adjusting the provided composition to the requirements of the 3D printing technology by means of general routine work normal in the field of 3D printing technology. The 3D printing process may be software controlled.

3D printing as used herein may refer to any printing technology in which material is joined or solidified under computer control to create a 3D object or structure, usually by successively adding material layer by layer. A particularly suitable example of 3D printing technology for use with the present invention is fused deposit modelling (FDM) which is described further below. However, other 3D printing methods and devices are available on the market or are in development. As such, it is understood that the skilled person will be capable of adjusting the herein described invention to be compatible with any 3D printing technology by means of general routine work in the field of 3D printing technology.

A particularly suitable example of 3D printing technology for use with the present invention is fused deposit modelling (FDM). FDM is typically regarded as the most low cost and easily accessible 3D printing technology. In FDM 3D printers, thermoplastic filaments are typically used as the starting material. In one aspect, the printable filaments according to the present invention may thus be used as starting material for FDM. The filaments are melted or softened, and then extruded from a printer head that is able to move in the x- and y-direction. Further, the printer head or the platform is able to move in the z-direction. The 3D object is formed by stacking several layers of filament. Each layer fuses and bonds to the layers below. The layers of melted filament solidify after being extruded from the print head. As one layer cools down and solidifies, another layer of the melted filament will be deposited on top of it until the 3D printing of the object is completed. Accordingly, if the 3D printing is FDM 3D printing, the method may comprise the steps of:

(i) Producing a 3D printable filament from the formulation as described herein, and

(ii) FDM 3D printing a pharmaceutical dosage form from the printable filament from step (i).

The FDM printing temperature may be in the range from 120 °C to 180 °C, preferably 130°C to 1160°C, more preferably about 150 °C; for example 148°C; for example 150°C. The preferred temperature also depends on the layer height as an increase of the layer height may entail a higher flow of matter which may require a higher temperature for the printing process.

Hot Melt Extrusion (HME) technology can be used to produce the filaments required for 3D printing using an FDM 3D printer. In some embodiments, HME is also used to produce the pre-formulation blend that comprises at least one thermoplastic polymer, such as the first and second thermoplastic polymer, and optionally at least one plasticizer. HME is a continuous process where heat and pressure are applied to melt or soften material through an orifice to produce thermoplastic filaments of uniform shape and density. The extruder may contain heaters that provide heat for the melting or softening of the materials. The screws in the extruder can provide shear stress and intense mixing of the materials. The friction created by the screws in the barrel and the heat provided cause the polymeric material to melt. The screw then conveys the melted material down the barrel for extrusion. Accordingly, if the 3D printing is FDM 3D printing, the method may comprise the steps of producing 3D printable filament from said formulation filament by means of HME; in particular when referring to step (i).

The HME printable filament may have a diameter of at least 1.5 mm to at most 3.1 mm, preferably 1.6 mm to 3.0 mm, more preferably 1.7 to 2.9 mm. In a particular embodiment, the HME printable filament may have a diameter of 1.50 mm to 1.90 mm, preferably 1.60 mm to at most 1.80 mm, more preferably 1.65 to 1.75 mm, even more preferably about 1.70 mm. In a particular embodiment, the HME printable filament may have a diameter of at least 2.65 mm to at most 3.05 mm, preferably 2.75 mm to at most 2.95 mm, more preferably 2.80 mm to at most 2.90 mm, even more preferably about 2.85 mm. The HME extruding temperature may be in the range from at least 140°C to at most 190°C, preferably 140°C to at most 160°C, even more preferably 142°C to at most 158 °C, even more preferably 144°C to at most 156°C, even more preferably about 150°C. The HME extrusion speed may be in the range from at least 5 to at most 15 rpm; preferably 6 to 14 rpm, more preferably 7 to 13 rpm, more preferably 8 to 12 rpm, even more preferably 9 to 11 rpm, even more preferably about 10 rpm.

In some embodiments, the use of the formulation or of the printable filament as disclosed herein, is thus envisaged for 3D printing; preferably for 3D printing by fused deposition modelling (FDM). More specifically, said use is for 3D printing, preferably FDM, of a modified-release dosage form. The modified-release dosage form can be a pharmaceutical dosage form, such as for example an implantable tablet or capsule, also called a printlet.

In another aspect, a method to prepare a printed pharmaceutical dosage form or a printlet, preferably a modified-release pharmaceutical dosage form or printlet is disclosed. Said method comprises melting and printing a formulation or a printable filament prepared according to any of the methods disclosed herein, wherein printing is performed with 3D printing, more specifically by fused deposition modelling (FDM).

In a further aspect of the application, the formulation, the printable filament or the pharmaceutical dosage form as disclosed herein or as prepared by any of the disclosed methods, is provided for use as a medicament. In some specific embodiments, and when the API is paliperidone palmitate, the formulation, the printable filament or the pharmaceutical dosage form as disclosed herein or as prepared by any of the disclosed methods is for use as a medicament for psychotic diseases, such as schizophrenia.

The use as medicament may be understood to refer to a method of treatment of a subject in need of an API comprising the steps of administering to a subject a pharmaceutical formulation, a pharmaceutical dosage form or a 3D printed pharmaceutical dosage form according to any of the embodiments as described herein.

In preferred embodiments, the pharmaceutical dosage form, preferably the 3D printed pharmaceutical dosage form is an implantable dosage form and will be implanted subcutaneously in the subject.

In some preferred embodiments of this application, the subject is a subject in need to receive a medical treatment. In some embodiments, the subject is a human subject.

It is apparent that there have been provided in accordance with the invention products, methods, and uses, that provide for substantial advantages as set forth above. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The above aspects and embodiments are further supported by the following non-limiting examples.

## EXAMPLES

### 1. Materials and methods

### 1.1 Materials

PLA 4060D, amorphous PLA with 12% D-Lactide, was purchased from Ingeo NatureWorks® (USA), EVA 233 with 23% of vinyl acetate content was purchased from Exxon Mobil Chemical Company®(USA). Paliperidone palmitate was purchased from Biochem Partner (China), Polyethylene glycol 2000 (PEG2000) was purchased from Sigma-Aldrich® (USA), Poloxamer® 188 (P188) was kindly donated by BASF® (Germany). Trifluoroacetic acid, chloroform, acetonitrile and isopropanol were purchased from Sigma-Aldrich® (USA). Hydrochloric acid and Tween® 20 were purchased from VWR® (USA).

**1.2 Preparation of Amorphous solid dispersions and evaluation**

*1.2.1 Hot-Melt Extrusion (HME)*

As PLA is usually extruded at a temperature of around 180°C, i.e., close to the degradation temperature of PP, the direct preparation of a polymer-excipient-API mixture cannot be carried out in a single step process. Therefore, it was necessary to plasticize PLA during a pre-formulation step. This first blending step was made using an internal blender (Brabender® model 50 EHT (Brabender GmbH&Co)). This step was performed on 40g in sample:

**Table 2: Different blends processed with the Brabender® - Temperature set at 180°C and the rotation speed kept at 150 RPM for 5 min**

| Formulations | PLA (%) w/w | EVA (%) w/w | P188 (%) w/w | PEG (%) w/w |
|---|---|---|---|---|
| PLA_P188 | 90 | - | 10 | - |
| PLA_PEG | 90 | - | - | 10 |
| PLA_EVA | 80 | 20 | - | - |

After this step, the resulting materials were coarsely ground with an IKA® A10 mill (Werke GmbH & Co, Staufen, Germany) to facilitate its milling.

*1.2.2 Cryomilling*

Cryogenic milling was conducted in an oscillatory Retsch® Cryomill (Retsch GmBH, Haan, Germany). The formulations were placed in a 25ml stainless steel grinding jar with 3 stainless steel beads of 15mm. The milling time was divided into different cycles of 2 minutes at 30Hz and different cycles of 30 seconds at 5Hz to avoid any overheating. The cryogenic milling was carried out on formulations to reduce the aggregates into powder. Then, formulations were passed through a 40mesh sieve.

*1.2.3 Preparation of drug loaded filament by melt extrusion*

Drug loaded filaments were prepared by HME using a parallel twin-screw extruder (Thermo Scientific® Process 11, Thermo Fisher Scientific Inc., USA) with 8 separate heating zones, including the die (ø = 1.70 mm). Temperature, die pressure, torque, and speed of rotation of the screws were continuously monitored. The speed of the screws was fixed at 30 rotations per minute (RPM). The temperature of the different heating zones was fixed as follows (from zone 1 to zone 8): 60/80/125/125/125/125/125/110°C. A volumetric feeder (Volumetric Mini Twin-Feeder, Thermo Fisher Scientific Inc., USA) was used to convey the formulations into the extruder and the screw speed of the feeder was set at 5rpm. After extrusion, the filaments were collected using a filament winder to obtain a diameter of $1.7 \pm 0.1$mm.

*1.2.4 Thermal analysis*

Differential scanning calorimetry (DSC) was conducted with a DSC Q2000 with $T_{zero}$ Technology and RCS cooling system. Temperature and enthalpy calibrations were performed using an indium standard (TA Instruments, New Castle, USA). Approximately 5-10mg of samples were sealed in $T_{zero}$ hermetic aluminum pan. The samples were heated from -50°C to 130°C with a heating rate of 10°C/min. All analyses were conducted under nitrogen atmosphere (50ml/min).
Thermal decomposition of samples was assessed by thermogravimetric analysis (TGA). The analysis was performed with a TGA Q500 (TA Instruments, New Castle, USA). Samples of 5-10 mg were loaded into a platinum pan and were heated from 30°C to 450°C with a heating rate of 10°C/min under nitrogen gas (flow rate: 60ml/min).

*1.2.5 Gel Permeation Chromatography*

Gel permeation chromatography (GPC) analysis was conducted on an Agilent liquid Chromatography (Agilent Technologies, United States) equipped with an Agilent degasser, an isocratic HPLC pump with a flow rate set at 1ml/min. Chloroform was used as mobile phase and polystyrene were used as standards for calibration. The GPC was equipped with an Agilent autosampler, the loop volume was 100μL. The solutions were concentrated at 2mg/ml. The GPC was equipped with an Agilent DRI refractive index detector and three columns: a PL gel 5mm guard column (Polymer Laboratories, Ltd, United Kingdom) and two PL gel Mixed-B 5μm columns (columns for separation of polystyrene with a Mw ranging from 200 to $4\times10^5$ g/mol) were used at 30°C to evaluate the Mw of samples.

*1.2.6 X-Ray Powder Diffraction*

A powder X-Ray diffractometer (D8 Advance Eco Bruker®, Madison, USA) equipped with a one-dimensional silicon detector (LynxEye, Bruker AXS) was used to characterize the crystalline/amorphous structure of PP, starting elements and formulations. Using a Cu K$\alpha$ radiation (1.54 Å; 40 kV $\times$ 25mA) data were collected, over the angular range of 3-45° 2$\theta$ and a step size of 0.02° and a dwell time of 1s.

*1.2.7 Determination of drug loading*

To extract the API, samples loaded with an average weight of 1mg of API were solubilized in 1 part of chloride methylene under vortex until its complete solubilization. Then, 9 parts of isopropanol were added drop by drop under vortex. Solutions were filtered through 0.22$\mu$m filters (Sortorius®) and filled in 2ml vial for HPLC analysis.

An HPLC-UV method was conducted to evaluate the drug loading from the extruded filaments as well as from 3D implants. Mobile phase A, which consisted of acetonitrile (100% v/v), and mobile phase B (aqueous solution of trifluoroacetic acid at pH 2) were used at ratio 70/30 A/B (v/v). The flow rate was set at 1ml/min for 20 min and the wavelength was fixed 278 nm. The retention time of PP was 8.0min.

*1.2.8 Dielectric spectroscopy*

Different formulations were studied using dielectric spectroscopy. The samples in the form of extruded films were placed between two brass electrodes and measured under an inert atmosphere of helium. Measurements of electric impedance were performed within the frequency range from 1Hz to 1MHz at different fixed temperatures, from 30°C to 140°C with a 2°C step, via a ModuLab XM MTS, Solartron Analytical® (Ametek, United-Kingdom). The sample temperature was precisely controlled using a cryostat and a proportional-integral-derivative (PID). The complex dielectric constant ($\varepsilon$), which is a function of frequency ($\omega$) and temperature (T), was analyzed using the empirical Havriliak-Negami (HN) (1) equation as given by:

$$\varepsilon(\omega, T) = \varepsilon_\infty + \left[ \frac{\Delta\varepsilon}{(1 + (i\omega\tau_{HN})^\alpha)^\beta} \right] \qquad (1)$$

where $\Delta\varepsilon$ and $\tau_{HN}$ are the dielectric strength and the HN characteristic time, $\varepsilon_\infty$ is the high frequency permittivity and $\tau$ is the relaxation time. $\alpha$ and $\beta$ are the shape parameters, respectively, related to the width and the asymmetry of the loss curves. The position of the maximum of the relaxation peak was, hence, obtained as

$$\omega_{max} = \frac{1}{\tau_{HN}} \left[ sin \frac{\alpha\pi}{2 + 2\beta} \right]^{\frac{1}{\alpha}} \left[ sin \frac{\alpha\beta\pi}{2 + 2\beta} \right]^{-\frac{1}{\alpha}} \qquad (2)$$

Via a model free approach, the segmental relaxation time $\tau$ is related to the maximum of the relaxation peak as $\tau\omega_{max}$ = 1. Glass transition temperature ($T_g$) was calculated by fitting the temperature evolution of relaxation time (r) using the Vogel-Fulcher-Tammann (VFT) equation (3), given by:

$$\tau = \tau_\infty exp \left( \frac{DT_0}{T - T_0} \right) \qquad (3)$$

where, $\tau_\infty$ and D are constants and $T_0$ is called Vogel temperature. Though it was not possible to follow the segmental process down to frequencies lower than 1 Hz, the dynamic glass transition temperature was obtained by extrapolating the temperature dependence of $\tau$ down to the temperature at which the segmental time reaches 100 s.

*1.2.9 Design software*

Tinkercad® was used as computer aided design (CAD) program to design the geometry of our implantable dosage forms. The generated CAD files were converted into .stl files. Then, the obtained files were imported into an open-source software for slicing before printing. Slic3r® 1.3.0, was used to generate .gcode files compatible with the 3D printer.

*1.2.10 3D Printing*

FDM was conducted on a Hyrel® system 30M (Norcross, USA), equipped with a MK1 head adapted to 1.7 mm diameter printable filament and a nozzle head of 0.4 mm diameter. The printing temperature was set at 150°C, the layer thickness at 0.2 mm, the print speed at 10 mm/s and the building plate was heated at 40°C.

*1.2.11 Dissolution test*

An Eppendorf ThermoMixer® C (Eppendorf AG, Germany) was used to perform the dissolution tests with the filaments. The temperature was set at 37°C and the rotation speed at 600 RPM. The filaments were placed in 2ml Eppendorf® and filled with 1.5ml of dissolution medium that was consisted of 2% (w/w) Polysorbate 20® in 0.001N HCl. For the 3D printed implants, the dissolution test was performed in a GFL® (Burgwedel, Germany) water bath kept at 37°C. Implants were placed in 20ml of dissolution medium. The *in vitro* dissolution tests of both filaments and implants were performed in triplicate and the medium was replaced at each sampling time to maintain sink conditions.

## 2. Results

### 2.1 Characterization of raw materials

Along the successive steps to get a final printable dosage form, multiple heating processes are often required and may have an adverse structural effect on API and its associated materials. For that reason, thermal analyses on starting materials were conducted to better understand the thermal stability of our different constituents during the HME and 3D printing processes. Thermal transitions of the different materials were first studied by DSC analyses.

The DSC curves in Figure 2 corresponded to the results obtained from the 2nd heating cycle. Indeed, a heating and a cooling ramp were applied to the different starting materials to eliminate their thermal history. The results showed that after a first heat/cool cycle, PP crystallized upon the second heating cycle with a cold crystallization peak at 56°C and a melting point at 114°C. EVA showed a wide melting range with a maximum at 50°C. For PLA4060D, only one single Tg was identified on the 2nd cycle at 59°C, in agreement with its amorphicity. PEG2000 and P188 presented a similar melting point at 55°C.

Then, a TGA was carried out on the different starting materials providing initial information about their thermal stability. This analysis provided the maximum values that should not be exceeded during thermal processes to avoid any thermomechanical degradation (e.g. HME or 3D printing).

The results obtained from the percentage weight loss and the first derivative of the different constituents, PLA, P188 and PEG, did not show any mass loss before 300°C (Figure 3). On the other hand, PP presented a lower thermal stability than the polymers as its weight loss started at 200°C. Indeed, a degradation step from PP could be observed between 200°C and 300°C, confirmed by its first derivative with a maximum peak at 300°C. In this case, 200°C was determined as the maximum temperature that should not be exceeded during our various thermal processes. In addition, none of the starting materials contained any residual moisture. However, TGA did not provide any chemical data about PP and its potential chemical degradation throughout the manufacturing processes. Indeed, other chemical processes might happen during the different processes, which may chemically induce the API degradation. Besides working at a temperature lower than 200°C, insights into any potential chemical instabilities or degradation of PP may be useful. For that reason, a liquid-liquid extraction followed by an HPLC-UV analysis was carried out after these thermal processes to evaluate the chemical integrity of the drug (see filaments and implant preparation). In order to evaluate the crystalline structure of PP as well as of the polymers before HME and FDM, XRD analysis were also made on these raw materials.

The XRD results of the different products are shown in Figure 4 and showed that neither PLA4060D or EVA233 presented any diffraction peaks. Poloxamer® 188 showed two characteristic peaks at 19° and between 22° and 23°. On the other hand, PEG 2000 showed four diffraction peaks at 13.5°, 19.1°, 23.2° and 27.2°. Two major peaks, at 5.1° and 7.7°, were observed for PP, corresponding to its crystalline structure. In comparison to other excipients diffractograms, PP was the only element presenting two major peaks between 3° and 10°. Based on that, these two peaks were used in the following part to highlight the presence of crystals PP residues in blends.

### 2.2 Pre-formulation blends

HME and 3D printing by FDM brought thermal stresses to the various components of the formulations. According to the TGA results (Figure 3), 200°C was the maximum temperature that PP could withstand. This temperature was close to the usual processing temperature of PLA, which is usually ranged between 180°C and 200°C[1,2]. Moreover, other parameters such as the screw design, the pressure and the residence time in the extruder may induce some degradation to API[3].

In order to decrease the processing PLA temperature, different types of plasticizers were used. Plasticizers are available in liquid or solid form. However, liquid plasticizers, such as citrate esters or triacetin, do not allow homogeneous mixing with powder constituents. In addition, evaporation or demixing of liquid plasticizers may lead to some stability issues in the final product[4]. In this work, the use of solid plasticizers was selected as an adequate solution. Indeed, both PEG 2000 and Poloxamer 188 were selected for their plasticizing effect and their current use in HME[4-7]. On the other hand, EVA was selected for its hydrophobicity, low processing temperature and its suitability for the preparation of subcutaneous implantable dosage forms, especially by HME[8]. The pre-formulation blends, consisting of PLA_PEG, PLA_P188, and PLA_EVA mixtures (Table 1), were prepared by HME. The process involved blending the mixtures at 180°C for 5 minutes using a Brabender® extruder. Then, the mixtures were cooled, grounded into a fine powder and sieved through a 40mesh sieve to remove any large agglomerates. Then, an extrusion test at a temperature slightly above the melting temperature of PP was conducted on the different blends. The selected temperature was 125°C, which was 10°C above the melting temperature of PP. Working at a temperature slightly above the melting point of PP prevented the screws from blocking during the HME process. All formulations were finally evaluated by DSC to determine whether the excipients had an impact on the Tg of PLA.

On the first heating cycle, the DSC results showed a decreased of the Tg to 43°C, 38°C and 54°C for the PLA_P188, PLA_PEG and PLA_EVA mixtures, respectively (Figure 5). The analysis was performed on the first heating cycle to obtain the Tg of the different formulations just after their preparation. Indeed, the characteristics seen in the first heating correspond to the state of the samples after extrusion without any further cooling or heating applied during the DSC analysis. Both P188 and PEG 2000 had a clear plasticizing effect on PLA4060D, decreasing the Tg of the polymer by 16°C and 21°C, respectively. On the other hand, EVA did not clearly affect the Tg of PLA4060D. Indeed, the presence of 20% w/w of EVA only decreased the Tg by 5°C. While 10% w/w of PEG or P188 decreased the Tg of PLA4060D, respectively by 36% or 27%. Despite this, all blends have been successfully extruded at 125°C without blocking the screws. In the case of PLA_EVA, the low melting temperature of EVA (55°C) and its presence at 20% w/w in the mixture resulted in a lower extrusion temperature compared to PLA4060D without additives. Then, the pre-formulations were ground by cryogenic milling and sieved to obtain a fine powder.

### 2.3 Filaments and implants preparation

Subsequently, PP was added to the various pre-formulations to achieve the described drug-loaded formulations (Table 3). After the addition of the drug, cryogenic milling was applied on the freshly prepared drug-loaded formulations. This milling step allowed the preparation of an homogeneous and intimate mixture of our different constituents[9]. After the milling step, the preparation of printable filaments from the mixtures was carried out. Through the HME process, the feeder, the screws and the winding speed were adjusted to control the filament diameter. The obtained filaments were characterized by a diameter of 1.7 ± 0.1mm which was suitable for the Hyrel® 3D printer. Then, the different filaments were analyzed by DSC and XRD. In order to assess the integrity of the drug after its thermal process, PP was extracted using a liquid-liquid extraction technique and quantified by a HPLC-UV method. Both the amount and integrity of PP were evaluated accordingly.

First, DSC analysis was performed on the different extruded filaments to highlight the different thermal transitions as well as the impact of PP on the pre-formulations blend. The DSC result, shown in Figure 6, showed different thermal transitions in the obtained filaments right after their preparation, during the first heating cycle. A decrease of the Tg was observed for PLA_P188_PP and PLA_PEG_PP mixtures. The observed values were 35°C and 32°C respectively for PLA_P188_PP and PLA_PEG_PP, compared to 43°C and 38°C in blends without PP.

For PLA_EVA_PP, the DSC results showed a Tg at 52°C, slightly lower than that obtained without PP. Moreover, the plasticizing effect of PEG and P188 on PLA4060D, an additional decrease of the Tg was observed. The presence of 10% w/w of PP allowed a slight additional decrease of Tg observed in all blends.

In addition to the Tg, cold crystallization peaks corresponding to PP were also observed at 62°C, 64°C and 80°C for PLA_P188_PP, PLA_PEG_PP and PLA_EVA_PP, respectively. These cold crystallization peaks appeared at higher temperatures than that for amorphous PP, which was of 56°C (Figure 2). The impact on the minimum temperature required to crystallize PP was related to the formulations. Indeed, the highest value was observed for PLA_EVA_PP with an increase of 24°C. On the other hand, a relatively low increase was noticed for the formulations based on PLA_P188 and PLA_PEG. In their work, *Sarpal et al,* prepared amorphous solid dispersion based on melt-quenched drug-loaded polymers. They observed an increase in the cold crystallization peak by DSC, which was correlated as an indicator of the strength of drug-polymer interaction[10]. These observations were also shared by *Bhugra et al.* who worked on the physical stability drug-loaded polymers system. They observed that higher cold crystallization temperatures were related to longer stability overtime[11].

Finally, the melting point of PP in the different blends was observed at 106°C, 107°C and 104°C for PLA_P188_PP, PLA_PEG_PP and PLA_EVA_PP, respectively. This melting point appeared to be 10°C lower than that from pure PP. A decrease of the melting point of a drug might be due to the miscibility of this drug in the blend. On the other hand, immiscible or partially miscible drug-polymer system would not show any melting point decrease [12]. In this case, the endotherm of PP was related to amorphous drug-rich nanodomains as the event was taking place right after a cold crystallization peak. Indeed, *Sarpal et al.* reported this phenomenon and suggested amorphous drug-rich domains can rearrange into crystalline phases correlated to an increased mobility of the system above Tg[10].

In order to complete these observations, XRD analysis was carried out throughout the various preparation steps of the printable filaments. The samples were analyzed before and after cryogenic milling, as well as after the extrusion of the filaments. This analysis highlighted the impact of these different processes on the PP state. Both cryogenic milling and HME process involved the application of mechanical and/or thermal energy to the various constituents of the blends.

Investigating the physical mixtures of PLA_P188_PP, PLA_PEG_PP and PLA_EVA_PP, before the cryogenic milling (Figure 7A), showed the different diffraction peaks of crystalline PP. Indeed, both characteristic peaks between 3° and 10°, at 5.1° and 7.7° from PP can be clearly highlighted regardless the formulation. In Figure 7B, i.e., after the cryogenic milling stage, a clear decrease in the intensity of PP diffraction peaks was observed. Without wishing to be bound by any hypothesis, the inventors propose that the mechanical stress involved during the milling step allowed an intimate mixing of the different constituents and a partial amorphization of the drug. Milling crystalline materials may lead to their amorphization by disrupting the crystalline lattice. However, the material is subjected to high energy milling and thus significant heat, that may induce some thermal degradation. Through the use of cryogenic milling, under liquid nitrogen, the excess of heat produced during the milling stage could be avoided[13].

On the other hand, after the final extrusion stage (Figure 7C), no diffraction peaks of PP were observed in the filaments, corresponding to its amorphous state which was in correlation to the DSC results. Without wishing to be bound by any hypothesis, the inventors propose that the thermal process, the shear force applied by the screws and the rapid cooling of the filament after extrusion completed up the amorphization of PP. However, the chemical degradation that may occur during cryogenic milling or HME process could not be assessed merely by DSC or XRD analyses. Therefore, the integrity and the quantification of PP was evaluated on the extruded filaments at the end of the process.

*Table 3: Composition of the different formulations*

| Formulations | PLA (% w/w) | EVA (% w/w) | P188 (% w/w) | PEG (% w/w) | Theoretical loading of PP (% w/w) | Experimental PP (% w/w) (mean ± SD, n=3) |
|---|---|---|---|---|---|---|
| PLA_PEG_PP | 81 | - | - | 9 | 10 | 9.6 ± 0.1 |
| PLA_ P188_PP | 81 | - | 9 | - | 10 | 9.8 ± 0.1 |
| PLA_EVA_PP | 72 | 18 | - | - | 10 | 9.8 ± 0.3 |

After our different processes, the percentages of PP after extraction from the filament in the different formulations were 9.6 ± 0.1%, 9.8 ± 0.1% and 9.8 ± 0.3% from PLA_P188_PP, PLA_PEG_PP and PLA_EVA_PP, respectively. In addition, no evidence of PP degradation was observed through these analyses. Based on these values, it could be concluded that the chemical stability PP was preserved through the different milling and extrusion stages.

However, before being printed, a first screening was performed on the different formulations. An *in vitro* dissolution test was carried out on different samples obtained from PLA_P188_PP, PLA_PEG_PP and PLA_EVA_PP filaments. The aim of this test was to assess the release of PP after 90 days from the different formulations. For this purpose, different samples of the formulations were placed in 2ml Eppendorf® and filled with 1.5ml of dissolution medium. During the *in vitro* dissolution test, the medium was replaced at each sampling time and analyzed by HPLC-UV to quantify the amount of PP released.

After 1 day of dissolution, no PP was released from the filaments based on PLA_P188_PP and PLA_PEG_PP formulations (Figure 8). On the other hand, 3.8 ± 0.3% of PP was released after 1 day of dissolution from the PLA_EVA_PP formulation. After 90 days of dissolution, only the PLA_EVA_PP based formulation showed a continuous and sustained release of PP with time to reach 22.9 ± 2.8% after 90 days. As a filament is a semi-finished product, the following part on 3D printing was carried out with the PLA_EVA_PP formulation which allowed a sustained release of the drug overtime.

To complete these observations, broadband dielectric spectroscopy (BDS) was used to characterize the molecular mobility of the different formulations studied in the *in vitro* dissolution test on filaments (Figure 9). Without wishing to be bound by any hypothesis, the polymer type and in this case the pre-formulation blend might have an impact on the molecular mobility within the filament. BDS is a suitable technique to monitor the molecular mobility as it displayed in relaxation processes over a wide range of frequency and at different temperatures[14].

Based on the temperature dependence of the segmental time within the interval from 30 to 85°C, PLA_EVA_PP showed a clear lower molecular mobility than PLA_P188_PP and PLA_PEG_PP. In addition, the extrapolated Tg from BDS evaluation (Table 4) were in agreement with those observed by DSC (Figure 6).

*Table 4: Comparison of Tg obtained by BDS and DSC.*

| Sample names | Tg (BDS) (°C) | Tg (DSC) (°C) |
|---|---|---|
| PLA-EVA-PP | 53 ± 1 | 52 |
| PLA-PEG-PP | 32 ± 1 | 32 |
| PLA-P188-PP | 35 ± 1 | 35 |

As already mentioned, at a temperature higher than $T_g$, an increase of the molecular mobility may enhance the crystallization ability[15] of the drug, and reduces the temperature at which cold crystallization takes place. Indeed, DSC measurements showed that the crystallization peak of PP appeared at a higher temperature for the filament based on PLA_EVA_PP mixture compared to the other formulations. Without wishing to be bound by any hypothesis, the inventors propose that, as the molecular mobility got reduced, it took a longer period of time for PP to crystallize. On the other hand, the PLA_P188_PP and PLA_PEG_PP formulations showed a $T_g$ being roughly 20 °C lower than PLA_EVA_PP, indicating an enhanced molecular mobility. During the *in vitro* dissolution test, the filaments were placed at 37°C for 3 months. Such temperature was higher than the $T_g$ of PLA_PEG_PP and PLA_P188_PP, which allowed a faster crystallization of PP and a significant reduction of its release from the corresponding filaments.

After this screening test, the filament based on the PLA_EVA_PP was selected and printed as an implant using the Hyrel® 30M 3D printer equipped with a MK-1 head. During the printing, the temperature was set at 150°C, the printing speed at 10mm/s and the heating plate at 40°C to ensure a good adhesion between the printed layer and the building plate. The shape of the implant has been designed via Tinkercad© (Figure 10).

On the market, subcutaneous implants are shaped as long cylinders. For instance, Viadur™ leuprolide acetate-loaded implant is designed as a reservoir-based system characterized by a diameter of 4mm and a length of 45mm[16]. Norplant™ is a contraceptive system that is loaded with levonorgestrel and measures 2.4mm in diameter and 4.4cm in length[17]. Lastly, Supprelin™ and Vantas™ are both subcutaneous implants based on histrelin acetate, characterized with a diameter of 3mm and 3.5cm in length[16].

3D printing is a highly versatile technology, allowing the conception of complex structures. In addition to the overall implant design, specific parameters applied in 3D printing may impact the surface of the final dosage form. In this work, a slightly flattened shape was designed to allow the modulation and evaluate the influence of the infill percentage on the release profile of the 3D printed implant. Indeed, a monolithic shape could be obtained with an infill of 100% while an infill equal to 0% corresponds to a hollow shape. The parameters that were selected to design our implants were 100% of infill (Figure 10, B left) and 20% of infill (Figure 10, B right). All implants were printed with a layer thickness of 200µm.

Following the 3D printing, the surface area and volume of the implants were determined using the Meshmixer© software (Table 4).

*Table 5: Table representing the theoretical and experimental surface (mm$^2$), volume (mm$^3$) and surface/ volume ratio (mm$^2$/mm$^3$) of the 3D implants*

| | Theoretical values | | | Experimental values (mean ± SD, n=3) | | | |
|---|---|---|---|---|---|---|---|
| Infill (%) | Surface (mm$^2$) | Volume (mm$^3$) | S/V (mm$^2$/mm$^3$) | Surface ± SD (mm$^2$) | Volume ± SD (mm$^3$) | S/V (mm$^2$/mm$^3$) | Weight (mg) |
| 20 | 474.2 | 188.7 | 2.5 | 491.9 ± 18.9 | 200.1 ± 11.7 | 2.5 ± 0.1 | 157.9 ± 6.2 |
| 100 | 316.1 | 281.2 | 1.1 | 314.1 ± 8.7 | 312.6 ± 16.8 | 1 ± 0.1 | 364.7 ± 14.7 |

When the printed device did not present any bottom or top layers, the infill percentage has a direct impact on the surface area and the volume of the final dosage form. In this case, decreasing the infill from 100%, corresponding to a monolith, to 20% increased the surface area of the implant. Indeed, the theoretical surface of the monolith which was evaluated with Meshmixer© was found to be 316.1 mm$^2$ and increased to 474.2 mm$^2$ when the infill was fixed at 20%. Nevertheless, decreasing the infill of the device also decreased its volume. The volume of the implant decreased from 281.2 mm$^3$ for a 100% of infill to 188.7 mm$^3$ for a 20% of infill. In addition, the surface-to-volume ratio of the implants was higher when an infill of 20% was set, compared that of the monolith.

The experimental values obtained with the 3D printed implants showed a surface area of 491.9 ± 18.9 mm$^2$

and 314.1 ± 8.7 mm$^2$ for the infill 20% and 100%, respectively. The obtained volume for the 20% and 100% implants were 200.1 ± 11.7 mm$^3$ and 312.6 ± 16.8 mm$^3$. The overall surface-to-volume ratio was maintained with a 2.5 ± 0.1 and 1 ± 0.1 mm$^2$/mm$^3$ ratio.

Even if the experimental volume of the monolith was 10% higher than expected, this value could be attributed to some slight differences in the 3D printing process. However, all the other values were within the standard deviations and the overall S/V ratio was preserved. The weights of the implants were of 157.9 ± 6.2 mg and 364.7 ± 14.7 mg for 20% and 100% of infill, respectively.

Individual implants were placed in glass flasks which were filled with 20ml of a similar dissolution medium as that used for our screening tests. The flasks were placed in an GFL bath, heated at 37°C with an orbital agitation of 50RPM for 90 days for the *in vitro* dissolution test. After each sampling time, the dissolution medium was replaced. The amount of PP that was released with time was quantified by an HPLC-UV method. The cumulative percentage of PP that was released from the implants with an infill of 20% and 100% as well as the obtained results of the filament are represented in Figure 11. In addition, GPC analysis was conducted on the PLA_EVA_PP filaments, on the 3D printed implants after 90 days *of in vitro* dissolution test.

After 1 day of *in vitro* dissolution test, the implants with 100% of infill released 2.5 ± 0.3 % of PP. On the other hand, the implant with an infill of 20% showed a higher release of PP which reached 6.3 ± 0.5% after 1 day. The implant with the largest surface area had the highest initial release percentage, in opposition with the monolith implant. At the end of the 3-month *in vitro* dissolution test, the implants based on 20% and 100% infill released 15.0 ± 0.8% and 5.6 ± 0.6% of PP, respectively. Therefore, the percentage of release was 2.7 times higher for the implants with a 20% infill compared to the monolith. In comparison, the filaments based on PLA_EVA_PP released 3.8 ± 0.3% of PP after 1 day and 22.9 ± 2.8% of PP after 90 days of dissolution test. In addition, based on GPC results (Figure 11, B), no degradation of the polymer was observed after the 3D printing process at 150°C in the presence of the drug. The slight differences in mass loss between the filament and the 3D printed implants were within the standard deviations. This is an important point because the presence of tertiary amine in the PP can be the starting point of amine-catalyzed hydrolysis and a faster degradation of PLA. Indeed, it has often been reported that risperidone in the presence of PLGA can accelerate the degradation rate of the polyester by the presence of amine associated with its piperidine ring[18-20]. In this study, neither HME or the 3D printing step caused a polymer matrix degradation. However, the presence of the piperidine ring in PP might increase the degradation rate of PLA during the dissolution test. Indeed, after 90 days of *in vitro* dissolution test, a clear mass loss was observed for both implants. Independently of the infill, the 3D printed implants presented an average mass loss of 45 ± 1% w/w. The decrease in Mw is explained by the degradation rate of the polymer succeeding the penetration of dissolution medium into the dosage form. The degradation of the polymer matrix is due to the hydrolytic cleavage of its ester linkages leading to the surface and bulk erosion of the 3D printed implant[21]. The differences of drug release could be thereby explained by the surface-to-volume ratio of the different implants. In fact, the implant with the infill of 20% showed a S/V ratio of 2.5 compared to 1.0 for the monolith. In comparison, the filaments used for the screening test presented a theoretical surface and volume of 36.4 mm$^2$ & 13.4 mm$^3$, respectively. These values corresponded to a surface-to-volume ratio of 2.7 mm$^2$/mm$^3$, which was still higher than that of the 20% infill implant. *Khaled et al.* worked on the geometry of the 3D printed acetaminophen-loaded tablets and showed that the surface area and the geometry had an impact on the drug release profile. They observed that the drug release from the 3D printed tablets was correlated to the S/V ratios, the higher the ratio was, the faster the drug released[22]. Therefore, the dissolution profile of PP obtained from the filament could be explained by its cylinder shape which allowed a higher S/V ratio than the 20% and 100% of infills. However, the results observed for the filament and the 20% infill implant showed a constant and sustained release of PP over 3 months in this study.

Nevertheless, these results showed that different and tailored released profiles might be obtained from a single formulation. In this case, no change has been made to the formulation, by only modulating the geometry of the final dosage form into different release profiles. Further studies with different infill range and geometries are conducted, especially the impact on the surface area, the volume and their S/V ratio are investigated. *In vivo* studies are conducted to complete the above observations. *Li et al.* compared the drug release from implantable devices made of drug-loaded PLA that did not release more than 2.5% of drug over 4 months *in vitro.* The same implants released up to 20% of drug within 30 days *in vivo. In vitro* dissolution test, or the latest particle-oriented dissolution method, approximates the complexity observed *in vivo,* but *in vivo* studies provide further valuable information, especially for long-acting implants.

## 2.4 Stability

Stability evaluations provide interesting information on how the quality of a drug varies with time under different conditions. In order to test the stability of PLA_EVA_PP formulation that was used in this study, 3D printed implants were prepared. The 3D printed dosage forms were placed in vials, flushed with nitrogen and sealed. Due to the presence of a polymer matrix, the implants must be stored at a low temperature[23]. For that reason, the vials were placed at 25°C and at 4°C.

XRD, drug extraction and GPC analysis were assessed during this stability study. XRD analysis was performed on 3D printed implants to assess the stability of the amorphous form of PP as a function of the storage and temperature. On the other hand, GPC analysis was performed after 3 months to investigate the stability of the polymer at 25°C and 4°C.

The results presented in Figure 12A and 12B showed the XRD analysis obtained during the stability evaluations. Figure 12A represented the XRD results from 3D printed dosage forms stored at 4°C after 1, 2 and 3 months of storage. An amorphous halo shape was observed at the different time and no crystal residue of PP was observed. Figure 12B showed the results obtained at a temperature of 25°C after 1, 2 and 3 months. The diffractograms did not show the presence of PP diffraction peaks, which correspond to crystals residues. The amorphous form of PP obtained after the different processes (mechanical and/or thermal) was maintained for at least 3 months at 4°C and 25°C. These observations were in correlation to those obtained by DSC and BDS (Table 2) as the storage temperatures were lower than the Tg of the PLA_EVA_PP formulation.

GPC evaluation allowed comparing the Mw of filaments that were freshly prepared and that of the implants after a storage of 3 months at 25°C and 4°C (Figure 12C). The results did not show any differences in mass loss between the samples, regardless of the storage temperature. In addition, the stability of PP in the 3D implants was assessed after 3 months. PP was extracted and quantified after being stored at 25°C and 4°C. The results showed a mean recovery of 97.3 $\pm$ 1.3% and 96.9 $\pm$ 1.4% after 3 months of storage, respectively at 4°C and 25°C. The stability of the drug was preserved, and no degradation was observed. These results agreed with an appropriate stability of 3D printed drug-loaded implants stored for 3 months at 4°C and 25°C.

## References

1. Boetker J, Water JJ, Aho J, Arnfast L, Bohr A, Rantanen J. Modifying release characteristics from 3D printed drug-eluting products. Eur J Pharm Sci. 2016;90:47-52. doi:10.1016/j.ejps.2016.03.013

2. Jamróz W, Kurek M, Czech A, Szafraniec J, Gawlak K, Jachowicz R. 3D printing of tablets containing amorphous aripiprazole by filaments co-extrusion. Eur J Pharm Biopharm. 2018;131:44-47. doi:10.1016/j.ejpb.2018.07.017

3. Huang S, Donnell KPO, Vaux SMD De, Brien JO, Stutzman J, Iii ROW. Processing Thermally Labile Drugs by Hot-Melt Extrusion: The Lesson with Gliclazide. Eur J Pharm Biopharm. 2017. doi:10.1016/j.ejpb.2017.05.014

4. Desai D, Sandhu H, Shah N, et al. Selection of Solid-State Plasticizers as Processing Aids for Hot-Melt Extrusion. J Pharm Sci. 2018;107(1):372-379. doi:10.1016/j.xphs.2017.09.004

5. Zhang Y, Luo R, Chen Y, Ke X, Hu D, Han M. Application of carrier and plasticizer to improve the dissolution and bioavailability of poorly water-soluble baicalein by hot melt extrusion. AAPS PharmSciTech. 2014;15(3):560-568. doi:10.1208/s12249-013-0071-x

6. Yu Y, Cheng Y, Ren J, Cao E, Fu X, Guo W. Plasticizing effect of poly(ethylene glycol)s with different molecular weights in poly(lactic acid)/starch blends. J Appl Polym Sci. 2015;132(16):1-9. doi:10.1002/app.41808

7. Lauer ME, Maurer R, Paepe AT De, et al. A Miniaturized Extruder to Prototype Amorphous Solid Dispersions : Selection of Plasticizers for Hot Melt Extrusion. Pharmaceutics. 2018;10(58). doi:10.3390/pharmaceutics10020058

8. Schneider C, Langer R, Loveday D, Hair D. Applications of ethylene vinyl acetate copolymers (EVA) in drug delivery systems. J Control Release. 2017;262(August):284-295. doi:10.1016/j.jconrel.2017.08.004

9. Allaf RM, Albarahmieh E, AlHamarneh BM. Solid-state compounding of immiscible PCL-PEO blend powders for molding processes. J Mech Behav Biomed Mater. 2019;97(February):198-211. doi:10.1016/j.jmbbm.2019.05.023

10. Sarpal K, Delaney S, Zhang GGZ, Munson EJ. Phase Behavior of Amorphous Solid Dispersions of Felodipine: Homogeneity and Drug-Polymer Interactions. Mol Pharm. 2019. doi:10.1021/acs.molpharmaceut.9b00731

11. Bhugra C, Telang C, Schwabe R, Zhong L. Reduced crystallization temperature methodology for polymer selection in amorphous solid dispersions: Stability perspective. Mol Pharm. 2016;13(9):3326-3333. doi:10.1021/acs.molpharmaceut.6b00315

12. Marsac PJ, Li T, Taylor LS. Estimation of Drug - Polymer Miscibility and Solubility in Amorphous Solid Dispersions Using Experimentally Determined Interaction Parameters. Pharm Res. 2009;26(1):40-44. doi:10.1007/s11095-008-9721-1

13. Moinuddin SM, Ruan S, Huang Y, et al. Facile formation of co-amorphous atenolol and hydrochlorothiazide mixtures via cryogenic-milling: Enhanced physical stability, dissolution and pharmacokinetic profile. Int J Pharm. 2017;532(1):393-400. doi:10.1016/j.ijpharm.2017.09.020

14. Grzybowska K, Adrjanowicz K, Paluch M. Application of Broadband Dielectric Spectroscopy to Study Molecular Mobility in Pharmaceutical Systems. Disord Pharm Mater. 2016:301-359.

15. Wang B, Mathew A, Napolitano S. Temperature and Thickness Dependence of the Time Scale of Crystallization of Polymers under 1D Confinement. ACS Macro Lett. 2021:476-480. doi:10.1021/acsmacrolett.1c00123

16. Simpson SM, Widanapathirana L, Su JT, et al. Design of a Drug-Eluting Subcutaneous Implant of the Antiretroviral Tenofovir Alafenamide Fumarate. Pharm Res. 2020;37(4). doi:10.1007/s11095-020-2777-2

17. Kleiner LW, Wright JC, Wang Y. Evolution of implantable and insertable drug delivery systems. J Control Release. 2014;181(1):1-10. doi:10.1016/j.jconrel.2014.02.006

18. Kohno M, Andhariya J V., Wan B, et al. The effect of PLGA molecular weight differences on risperidone release from microspheres. Int J Pharm. 2020;582(April):1-8. doi:10.1016/j.ijpharm.2020.119339

19. Rawat A, Stippler E, Shah VP, Burgess DJ. Validation of USP apparatus 4 method for microsphere in vitro release testing using Risperdal® Consta ®. Int J Pharm. 2011;420(2):198-205. doi:10.1016/j.ijpharm.2011.08.035

20. Shen J, Lee K, Choi S, Qu W, Wang Y, Burgess DJ. A reproducible accelerated in vitro release testing method for PLGA microspheres. Int J Pharm. 2016;498(1-2):274-282. doi:10.1016/j.ijpharm.2015.12.031

21. Bode C, Kranz H, Fivez A, Siepmann F, Siepmann J. Often neglected: PLGA/PLA swelling orchestrates drug release: HME implants. J Control Release. 2019;306(May):97-107. doi:10.1016/j.jconrel.2019.05.039

22. Khaled SA, Alexander MR, Irvine DJ, et al. Extrusion 3D Printing of Paracetamol Tablets from a Single Formulation with Tunable Release Profiles Through Control of Tablet Geometry. AAPS PharmSciTech. 2018;19(8):3403-3413. doi:10.1208/s12249-018-1107-z

23. Andhariya J V., Shen J, Wang Y, Choi S, Burgess DJ. Effect of minor manufacturing changes on stability of compositionally equivalent PLGA microspheres. Int J Pharm. 2019;566(June):532-540. doi:10.1016/j.ijpharm.2019.06.014

**Claims**

1. A method for preparing a formulation comprising an active pharmaceutical ingredient (API), said method comprising: melting a mixture comprising the API, at least one thermoplastic polymer and optionally at least one plasticizer, at a temperature at which the API is substantially not decomposed.

2. The method of claim 1, wherein the mixture comprises a first thermoplastic polymer and a second thermoplastic polymer, wherein the melting temperature of the second thermoplastic polymer is lower than that of the first thermoplastic polymer, and whereby the melting temperature of the mixture is lower than that of the first thermoplastic polymer.

3. The method of claim 1 or 2, wherein the mixture comprises the API and a pre-formulation blend comprising the at least one thermoplastic polymer and optionally the at least one plasticizer;

   preferably wherein the pre-formulation blend has been obtained by melting and allowing to solidify a mixture comprising the at least one thermoplastic polymer and optionally the at least one plasticizer;
   more preferably, wherein the pre-formulation blend has been obtained by hot melt extrusion (HME) of a mixture comprising the at least one thermoplastic polymer and optionally the at least one plasticizer.

4. The method of any one of claims 1 to 3, further comprising allowing the melted mixture comprising the API and the at least one thermoplastic polymer and optionally the at least one plasticizer, or comprising the API and the pre-formulation blend, to solidify,
preferably wherein the melted mixture has solidified into a filament, more preferably into a printable filament.

5. The method of any one of claims 1 to 4 wherein melting of the mixture comprising the API and the at least one thermoplastic polymer and optionally the at least one plasticizer, or comprising the API and the pre-formulation blend, is performed at a temperature at least 20°C lower, preferably at least 50°C lower, than the temperature at which the API begins to decompose, and/or at a temperature at most 20°C, preferably at most 10°, higher than the melting temperature of the API.

6. The method of any one of the preceding claims, wherein the at least one thermoplastic polymer comprises a polyester, preferably a biodegradable polyester, more preferably poly(lactic) acid (PLA), such as amorphous PLA.

7. The method of any one of the preceding claims, wherein the at least one thermoplastic polymer comprises ethylene vinyl acetate (EVA), preferably comprises EVA and a further thermoplastic polymer having melting temperature higher than EVA.

8. The method of any one of the claims 1 to 7, wherein the plasticizer is a polyether or stearic acid, preferably a polyether, such as wherein:

the plasticizer is selected from the group consisting of polyethylene glycol (PEG), poloxamer (poly(ethylene glycol)-*block*-poly(propylene glycol)-*block*-poly(ethylene glycol)), stearic acid, and mixtures thereof, or
the plasticizer is selected from the group consisting of PEG, poloxamer, and mixtures thereof, more preferably wherein:

the plasticizer is selected from the group consisting of polyethylene glycol 2000 (PEG2000), Poloxamer 188, poloxamer 407, stearic acid, and mixtures thereof, or
the plasticizer is selected from the group consisting of PEG2000, Poloxamer 188, and mixtures thereof.

9. The method of any one of the preceding claims, wherein the mixture comprises the API and:

PLA and EVA, preferably 60 wt% to 75 wt% of PLA in combination with 15 wt% to 30 wt% of EVA based on the total weight of the mixture (w/w);
PLA and a PEG, preferably PEG2000, preferably 70 wt% to 85 wt% of PLA in combination with 5 wt% to 20 wt% of PEG based on the total weight of the mixture (w/w): or
PLA and a poloxamer, preferably Poloxamer 188, preferably 70 wt% to 85 wt% of PLA in combination with 5 wt% to 20 wt% of poloxamer based on the total weight of the mixture (w/w).

10. The method of any one of the preceding claims, wherein the API is present in an amount of between 5 wt% and 15 wt% of the total weight of the mixture (w/w).

11. The method of any one of the preceding claims wherein the API is paliperidone palmitate (PP).

12. The method of any one of the preceding claims, wherein the melted mixture has solidified into a printable filament, further comprising modelling the printable filament into a pharmaceutical dosage form, preferably by fused deposition modelling (FDM), such as where the pharmaceutical dosage form is a printlet, such as an implantable modified release printlet.

13. A formulation prepared according to the method of any one of the preceding claims.

14. A printable filament comprising a homogeneous mixture of an API, at least one thermoplastic polymer and optionally at least one plasticizer, wherein the filament is configured to melt at a temperature at which the API is substantially not decomposed.

15. A pharmaceutical dosage form, preferably a 3D printed pharmaceutical dosage form, even more preferably a 3D printed modified release pharmaceutical dosage form, made from the formulation of claim 12 or from the printable filament of claim 13.

16. The formulation of claim 12, the printable filament of claim 13 or the pharmaceutical dosage form of claim 14 for use as a medicament.

17. A method to prepare a printlet; preferably a modified-release printlet, said method comprising melting and printing the formulation of claim 13 or the printable filament of claim 14; preferably wherein printing is performed with 3D printing; even more preferably wherein 3D printing is performed by fused deposition modelling (FDM).

Fig. 1

Fig. 2

## Fig. 3

## Fig. 4

Fig. 5

Fig. 6

Fig. 7

Fig. 8

Fig. 9

Fig. 10

Fig. 11

Fig. 12

Europäisches Patentamt
European Patent Office
Office européen des brevets

# EUROPEAN SEARCH REPORT

Application Number

EP 21 20 9513

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MANINI GIUSEPPE ET AL: "Long-acting implantable dosage forms containing paliperidone palmitate obtained by 3D printing", INTERNATIONAL JOURNAL OF PHARMACEUTICS, vol. 603, 12 May 2021 (2021-05-12), page 120702, XP055918820, NL ISSN: 0378-5173, DOI: 10.1016/j.ijpharm.2021.120702 | 1-6, 10-17 | INV. A61K9/00 B33Y10/00 B33Y70/00 A61K31/519 A61P25/18 |
| Y | * abstract * * page 1, right-hand column * * page 5, right-hand column * ----- | 7-9 | |
| Y | FENG XIN ET AL: "Twin-screw extrusion of sustained-release oral dosage forms and medical implants", DRUG DELIVERY AND TRANSLATIONAL RESEARCH, SPRINGER, GERMANY, vol. 8, no. 6, 12 December 2017 (2017-12-12), pages 1694-1713, XP036893193, ISSN: 2190-393X, DOI: 10.1007/S13346-017-0461-9 [retrieved on 2017-12-12] * figure 8 * * table 4 * * page 1694 * * page 1700, right-hand column * * page 1703 – page 1706 * ----- | 7-9 | TECHNICAL FIELDS SEARCHED (IPC) A61K B33Y A61P |

The present search report has been drawn up for all claims

| Place of search | Date of completion of the search | Examiner |
|---|---|---|
| Munich | 9 May 2022 | Vázquez Lantes, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................

& : member of the same patent family, corresponding document

EPO FORM 1503 03.82 (P04C01)

# EP 4 183 384 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOETKER J ; WATER JJ ; AHO J ; ARNFAST L ; BOHR A ; RANTANEN J.** Modifying release characteristics from 3D printed drug-eluting products. *Eur J Pharm Sci.,* 2016, vol. 90, 47-52
- **JAMRÓZ W ; KUREK M ; CZECH A ; SZAFRANIEC J ; GAWLAK K ; JACHOWICZ R.** 3D printing of tablets containing amorphous aripiprazole by filaments co-extrusion. *Eur J Pharm Biopharm.,* 2018, vol. 131, 44-47
- **HUANG S ; DONNELL KPO ; VAUX SMD DE ; BRIEN JO ; STUTZMAN J ; LII ROW.** Processing Thermally Labile Drugs by Hot-Melt Extrusion: The Lesson with Gliclazide. *Eur J Pharm Biopharm.,* 2017
- **DESAI D ; SANDHU H ; SHAH N et al.** Selection of Solid-State Plasticizers as Processing Aids for Hot-Melt Extrusion. *J Pharm Sci.,* 2018, vol. 107 (1), 372-379
- **ZHANG Y ; LUO R ; CHEN Y ; KE X ; HU D ; HAN M.** Application of carrier and plasticizer to improve the dissolution and bioavailability of poorly water-soluble baicalein by hot melt extrusion. *AAPS PharmSciTech,* 2014, vol. 15 (3), 560-568
- **YU Y ; CHENG Y ; REN J ; CAO E ; FU X ; GUO W.** Plasticizing effect of poly(ethylene glycol)s with different molecular weights in poly(lactic acid)/starch blends. *J Appl Polym Sci,* 2015, vol. 132 (16), 1-9
- **LAUER ME ; MAURER R ; PAEPE AT DE et al.** A Miniaturized Extruder to Prototype Amorphous Solid Dispersions : Selection of Plasticizers for Hot Melt Extrusion. *Pharmaceutics,* 2018, vol. 10 (58
- **SCHNEIDER C ; LANGER R ; LOVEDAY D ; HAIR D.** Applications of ethylene vinyl acetate copolymers (EVA) in drug delivery systems. *J Control Release,* August 2017, vol. 262, 284-295
- **ALLAF RM ; ALBARAHMIEH E ; ALHAMARNEH BM.** Solid-state compounding of immiscible PCL-PEO blend powders for molding processes. *J Mech Behav Biomed Mater,* February 2019, vol. 97, 298-211
- **SARPAL K ; DELANEY S ; ZHANG GGZ ; MUNSON EJ.** Phase Behavior of Amorphous Solid Dispersions of Felodipine: Homogeneity and Drug-Polymer Interactions. *Mol Pharm,* 2019

- **BHUGRA C ; TELANG C ; SCHWABE R ; ZHONG L.** Reduced crystallization temperature methodology for polymer selection in amorphous solid dispersions: Stability perspective. *Mol Pharm,* 2016, vol. 13 (9), 3326-3333
- **MARSAC PJ ; LI T ; TAYLOR LS.** Estimation of Drug - Polymer Miscibility and Solubility in Amorphous Solid Dispersions Using Experimentally Determined Interaction Parameters. *Pharm Res,* 2009, vol. 26 (1), 40-44
- **MOINUDDIN SM ; RUAN S ; HUANG Y et al.** Facile formation of co-amorphous atenolol and hydrochlorothiazide mixtures via cryogenic-milling: Enhanced physical stability, dissolution and pharmacokinetic profile. *Int J Pharm,* 2017, vol. 532 (1), 393-400
- **GRZYBOWSKA K ; ADRJANOWICZ K ; PALUCH M.** Application of Broadband Dielectric Spectroscopy to Study Molecular Mobility in Pharmaceutical Systems. *Disord Pharm Mater,* 2016, 301-359
- **WANG B ; MATHEW A ; NAPOLITANO S.** Temperature and Thickness Dependence of the Time Scale of Crystallization of Polymers under 1D Confinement. *ACS Macro Lett,* 2021, 476-480
- **SIMPSON SM ; WIDANAPATHIRANA L ; SU JT et al.** Design of a Drug-Eluting Subcutaneous Implant of the Antiretroviral Tenofovir Alafenamide Fumarate. *Pharm Res,* 2020, vol. 37 (4
- **KLEINER LW ; WRIGHT JC ; WANG Y.** Evolution of implantable and insertable drug delivery systems. *J Control Release,* 2014, vol. 181 (1), 1-10
- **KOHNO M ; ANDHARIYA J V. ; WAN B et al.** The effect of PLGA molecular weight differences on risperidone release from microspheres. *Int J Pharm,* April 2020, vol. 582, 1-8
- **RAWAT A ; STIPPLER E ; SHAH VP ; BURGESS DJ.** Validation of USP apparatus 4 method for microsphere in vitro release testing using Risperdal® Consta. *Int J Pharm.,* 2011, vol. 420 (2), 198-205
- **SHEN J ; LEE K ; CHOI S ; QU W ; WANG Y ; BURGESS DJ.** A reproducible accelerated in vitro release testing method for PLGA microspheres. *Int J Pharm,* 2016, vol. 498 (1-2), 274-282
- **BODE C ; KRANZ H ; FIVEZ A ; SIEPMANN F ; SIEPMANN J.** Often neglected: PLGA/PLA swelling orchestrates drug release: HME implants. *J Control Release,* May 2019, vol. 306, 97-107

- **KHALED SA ; ALEXANDER MR ; IRVINE DJ et al.** Extrusion 3D Printing of Paracetamol Tablets from a Single Formulation with Tunable Release Profiles Through Control of Tablet Geometry. *AAPS PharmSciTech,* 2018, vol. 19 (8), 3403-3413

- **ANDHARIYA J V. ; SHEN J ; WANG Y ; CHOI S ; BURGESS DJ.** Effect of minor manufacturing changes on stability of compositionally equivalent PLGA microspheres. *Int J Pharm,* June 2019, vol. 566, 532-540